# EUROPEAN PATENT APPLICATION

(11) **EP 4 817 447 A1**
(43) Date of publication of application: **07.10.2026**
(21) Application number: 26150515.0
(22) Date of filing: 07.04.2021
(51) Int. Cl.: A24F 42/20, A61M 15/06, A61M 11/00, A61M 15/00, A24F 40/05, A47J 43/12, A61K 9/12, A23P 30/40, A61P 11/00, A61P 15/10, A61P 25/26, B05B 7/00, A23L 2/40, A61K 9/00, A61K 31/352, A61K 31/465, A61M 16/00, A24F 40/10

(54) **FOAM CONSUMPTION DEVICE**

(30) Priority: 07.04.2020 GB 202005160; 28.05.2020 EP 20177196; 08.10.2020 WO PCT/EP2020/078344
(62) Document number(s) of the earlier application(s) in accordance with Art. 76 EPC: 21720970.9 / 4 117 472
(71) Applicant: Splash tm GmbH, 49733 Haren (DE)
(72) Inventor: HAGEN, Klaus, 49733 Haren (DE)
(74) Representative: Cyrson, Matthew Dominic

(57) **Abstract**

A battery-operated foam inhalation device which comprises a cartridge (610) having a liquid for producing a foam and a foam outlet (654). Agitation means agitates the liquid to produce the foam, and the agitation means comprises an electric motor (762) and a battery (760) for energising the electric motor (762). A carrier (672) receives the cartridge (610) and has a carrier foam inlet (680) for fluid communication with the foam outlet (654) of the cartridge (610). A carrier foam outlet (682) dispenses the foam to a user.

## Description

The present invention relates to an inhalation device. More particularly, the invention relates to a foam inhalation device, and especially a battery-operated foam inhalation device. The invention further relates to a foam drawing device for a user to draw foam therefrom with a user's breath and a cigarette substitute or alternative. Although described as an inhalation device, the device may also be considered to be a consumption device.

Inhalation devices typically are for users to inhale or consume substances or compounds. Such substances may have a variety of effects on the user, for example medicinal, therapeutic, stimulatory, relaxative or pleasurable effects. For example, a user may inhale medicines or tobacco components such as nicotine.

Traditional techniques for consuming or inhaling compounds such as nicotine rely on burning tobacco and either directly inhaling the smoke produced therefrom, for example as in cigarettes, or inhaling the smoke having passed through water, as in shisha or hookah. However, health concerns regarding smoke inhalation have created a need for non-smoking alternatives for inhaling compounds.

Typical alternatives include vaporisers or e-cigarettes. These involve heating a substance until it forms a vapour. However, health concerns have been raised regarding vaporisers.

Medical inhalers are known. However, such inhalers rapidly propel an aerosol into a user's respiratory system, and therefore provide a significantly different experience to smoking, which is done gradually and repeatedly by a user inhaling through a device over a prolonged duration.

It would therefore be desirable to provide a means of producing a substance for consumption or inhalation, and which can be drawn via a user's breath over a prolonged period, without requiring the use of a vaporiser or burning. Such an alternative may also find application in pharmaceutical, health and exercise fields, as well as the food and beverage industry.

The present invention seeks to provide a solution to these problems.

According to a first aspect of the invention, there is provided a battery-operated foam inhalation device comprising: a cartridge having a liquid for producing a foam, and a cartridge foam outlet; agitation means for agitating the liquid to produce the foam, the agitation means comprising an electric motor and a battery for energising the electric motor; and a carrier having a receiving portion for receiving the cartridge at or in the carrier, a carrier foam inlet for fluid communication with the foam outlet of the cartridge when the cartridge is received at or in the receiving portion, a carrier foam outlet for dispensing the foam to a user, and a conduit between the carrier foam inlet and the carrier foam outlet.

The device allows a user to draw foam with their breath into their mouth and then consume the foam. The foam is long-lived or stable, and so the user can draw the foam over a long duration. This provides an alterntive or replacement for smoking cigarettes, since it may be consumed in a similar way to cigarettes. The agitation means allows for a mechanical generation of foam which can be electrically powered. This allows for repeatable and controllable generation of foam, as and when it is required by a user.

Preferably, the device may further comprise a one-way valve for permitting movement of foam from the cartridge foam outlet to the carrier foam outlet and preventing or limiting movement of fluid from the carrier foam outlet to the cartridge foam outlet. This may prevent or limit the user from disrupting the foam in the device if the user blows back into the device.

Advantageously, the device may further comprise a pressure sensor for detecting when negative pressure is applied to the carrier foam outlet.

Beneficially, the pressure sensor may be communicatively connected with the agitation means so that when the pressure sensor detects negative pressure the agitation means is activated so as to agitate the liquid to produce foam. This allows for convenient generation of foam. The user is also not required to press a separate button, for example, to operate the agitation means which may provide a user-experience more comparable to traditional smoking.

In a preferable embodiment, the carrier may further comprise a carrier air inlet. The carrier air inlet may allow for the drawing of air from the exterior of the device to assist with foam generation.

Optionally, the cartridge may comprise a cartridge air inlet, the carrier comprises an air-flow conduit from the carrier air inlet for connection with the cartridge air inlet, and the agitation means comprises an air moving means driven by the electric motor for moving air from the air inlet to the cartridge. Such an arrangement allows for the movement of air from the exterior of the device to the cartridge for mixing with or bubbling through the liquid which generates the foam.

Additionally, the air moving means may comprise an air pump.

Preferably, the cartridge may comprise an air-injection conduit which extends from the cartridge air inlet into an interior of the cartridge for injecting air into the cartridge via at least one opening in the air-injection conduit. This can allow for a better generation of foam since liquid away from the cartridge air inlet can be exposed to the air. This may prevent or limit pockets of un-agitated liquid remaining in the cartridge.

The air injection conduit is here mounted to or is part of the cartridge. However, it will be appreciated that it may in fact be part of the carrier and extend into the cartridge via the cartridge air inlet.

Advantageously, the air injection conduit may have a plurality of openings therein. More openings may permit for faster generation of foam since more liquid can be exposed to the air.

Additionally, there may be at least five openings.

Beneficially, the air injection conduit may be elongate and the plurality of openings are spaced apart along a longitudinal extent thereof. A more uniform generation of foam may therefore be possible.

Preferably, said at least one opening may be closer to an end of the cartridge which opposes the cartridge air inlet than to the cartridge air inlet. This may further allow better generation of foam and may further prevent or limit pockets of un-agitated liquid remaining in the cartridge.

Optionally, each of the cartridge foam outlet and the cartridge air inlet may comprise a seal, at least part of the conduit of the carrier may be configured to perforate the seal of the cartridge foam outlet, and at least part of the air-flow conduit may be configured to perforate the seal of the cartridge air inlet. The seals allow for the cartridge to remain fluid-tight during transit, and the conduits being configured to perforate the seals allow for convenient fluid connection between the cartridge and conduit when required.

Advantageously, the conduit of the carrier and the air-flow conduit project into the receiving portion so as to permit perforation of the cartridge foam outlet and the cartridge air inlet respectively.

In a preferable embodiment, each seal is flexible so as to seal the cartridge foam outlet to the conduit of the carrier, and so as to seal the cartridge air inlet to the air-flow conduit. This may assist with preventing leaks from the cartridge.

Optionally the device may have a cartridge-presence sensor configured to detect when the cartridge is received in the cartridge receiving portion, the agitating means configured to operate only when the cartridge-presence sensor detects the cartridge in the cartridge receiving portion. As such, unintended operation of the agitations means may be prevented or limited which may reduce draining of the battery. Such a sensor may also be termed a switch.

Preferably, the liquid may further comprise at least one of nicotine, a component of cannabis, medicines and/or dietary supplements, and a food or drink substance.

Preferably, the device is elongate and has a length of 150 mm or less and a width of 50 mm or less. Such a sizing may allow for convenient holding and use of the device, and may provide a more comparable user-experience to cigarette smoking.

According to a second aspect of the invention there is provided a foam drawing device for a user to draw foam therefrom with breath of the user, the foam drawing device comprising: a container for containing a liquid for producing a foam, the container having a container foam outlet for emitting foam and a container air inlet for receiving air; an electrically energisable air moving means for moving air to the container air inlet from an exterior of the device; a power source for powering the electrically energisable air moving means; a mouthpiece fluidly communicated with the foam outlet for a user to draw foam from; a pressure sensor fluidly communicated with the mouthpiece for detecting when negative pressure is applied to the mouthpiece; a processor configured to operate the electrically energisable air moving means when the pressure sensor detects when negative pressure is applied to the mouthpiece.

According to a third aspect of the invention there is provided a foam inhalation device comprising: a cartridge having a liquid for producing a foam, and a cartridge foam outlet; an agitation means for agitating the liquid to produce foam; and a carrier having a receiving portion for receiving the cartridge at or in the carrier, a carrier foam inlet for fluid communication with the foam outlet of the cartridge when the cartridge is received at or in the receiving portion, a carrier foam outlet for dispensing the foam to a user, a conduit between the carrier foam inlet and the carrier foam outlet; and a carrier air inlet.

According to a fourth aspect of the invention, there is provided a stable-foam inhalation device for dispensing a stable foam to be inhaled by a user over a prolonged duration, the inhalation device comprising: a foam-generating-component receiving portion; stable-foam-generating components for generating the stable foam, said components being receivable at or in the component receiving portion, at least one of said components being segregated from another of said components by an openable barrier; an outlet for dispensing the stable foam to the user; a fluid flow path which fluidly communicates the foam-generating-component receiving portion with the outlet so that when the barrier is opened the components interact to generate the stable foam which flows to the outlet via the fluid flow path to be inhaled by the user over a prolonged duration.

A stable foam, as opposed to only a short-lived effervescence and/or a rapidly propelled inhalant, may allow for a user to consume or inhale a substance contained within the foam slowly, over a prolonged duration and with multiple inhalations. This can provide a satisfying and pleasurable experience for the user. The barrier maintains separation between the components until the user wishes to inhale foam, at which point the barrier may be broken to permit generation of foam.

The foam-generating-component receiving portion may otherwise be referred to as a foam-generating-component receiver, or a port, dock or a chamber.

Preferably, at least one of the stable-foam-generating components may be received in a non-electrical inhalation cartridge. A non-electrical inhalation cartridge reduces or eliminates a requirement for electrical charging. A cartridge provides a convenient way of loading stable-foam-generating components into the device, and permitting reuse of the remainder of the device with further cartridges.

Beneficially, at least one of the stable-foam-generating components may comprise a liquid which is received in a liquid-receiving chamber of the non-electrical inhalation cartridge.

Advantageously, the liquid may be dispensable from the liquid-receiving chamber via manual pressure applied to the chamber. Manual dispensation is convenient and prevents or limits the requirement for complex mechanisms.

In a preferable embodiment, at least one of the stable-foam-generating components may comprises a solid which is received in a solid-receiving chamber of the non-electrical inhalation cartridge.

Additionally, the openable barrier may be between the solid-receiving chamber and the liquid-receiving chamber.

Preferably, there may be two stable-foam-generating components, each stable-foam-generating component being received in a separate chamber, one of the chambers being on a path between the other chamber and the outlet so that the stable-foam-generating components mix in said other chamber. Therefore, the stable-foam-generating components are encouraged to mix which reduces the amount of unreacted material and thus reduces wastage.

Advantageously, the cartridge may include a further openable barrier at or adjacent to an end thereof and fluidly communicable with the fluid flow path so that when the further openable barrier is opened, foam or at least one stable-foam-generating component are flowable to the fluid flow path. The further barrier may help to contain the stable-foam-generating components when the cartridge is not inserted into the carrier.

Beneficially, the or each openable barrier may be a planar seal.

In a preferable embodiment, the or each openable barrier may comprise a foil. A foil, such as a metal foil, can provide a good seal between the components to maintain a long storage lifetime of the device, whilst being easily perforable or openable when required.

Optionally, the outlet and at least part of the fluid flow path may be defined by a carrier for the non-electrical inhalation cartridge.

Preferably, the fluid flow path may comprise a conduit at or adjacent to the outlet and an expansion chamber between the foam-generating-component receiving portion and the conduit. An expansion chamber provides space for the foam to expand into, and thus may allow for short-term storage of the foam before being inhaled.

Advantageously, the expansion chamber may be defined by the carrier. Since the expansion chamber would not be part of the disposable cartridge, wastage is reduced.

In a preferable embodiment, the conduit may have an inlet opening which is off-centre relative to the expansion chamber. Such an arrangement allows for the inlet opening to be at an in use lower position. Therefore, as the foam is consumed and a level of foam reduces in the expansion chamber, a greater total amount of foam can be accessed since the remaining foam would occupy the lower portion of the expansion chamber under gravity.

Beneficially, the conduit may have an inlet opening and a longitudinal extent which locates the inlet opening closer to the foam-generating-component receiving portion than to the outlet. This allows for foam to be inhaled away from an end of the cartridge. In other words, the foam may be drawn to the outlet from a more central region of the expansion chamber, which may improve the consistency of foam at the outlet.

Preferably, the device may further comprise a protrusion for assisting with dispensation of one of the stable-foam-generating components. The protrusion may be pointed, and thus may be considered to be a needle, which may assist with perforation of the barrier.

Additionally, the protrusion may be for perforating the or each openable barrier, the protrusion including a passageway for permitting passage of foam or stable-foam-generating component therethrough. A passageway, such as a bore or channel, prevents the protrusion from blocking the hole or perforation formed by the protrusion.

Optionally, the protrusion may be configured to perforate both openable barriers.

Advantageously, there may be two protrusions, a second protrusion configured to project into one chamber, and a first protrusion configured to project into at least the other chamber. These protrusions may be spaced apart, which permits for more efficient mixing of the components.

Beneficially, the device may further comprise a one-way valve for permitting movement of foam from the foam-generating-component receiving portion to the outlet and preventing or limiting movement of fluid from the outlet to the foam-generating-component receiving portion.

In a preferable embodiment, the stable-foam-generating components may together comprise a carbonate and an acid. This allows for effervescence by producing carbon dioxide via a chemical reaction. A chemical reaction, as opposed to, for example, releasing a pressurised container, is more convenient for manufacturing purposes. Although a carbonate is described, more generally the stable-foam-generating components may together comprise a base and an acid. The stable-foam-generating components may alternatively comprise any chemical gas generation means, in particular carbon dioxide generation means. It will be appreciated that the stable-foam-generating components may, for example, comprise a sugar and a component for converting the sugar to carbon dioxide, such as yeast.

Additionally, at least one of the carbonate and acid may be a powder. A powder has a larger surface are than other solids and therefore may more rapidly generate foam.

Preferably, the barrier may be opened by being broken.

Beneficially, one of the stable-foam-generating components may comprise a stabiliser.

Additionally, one of the stable-foam-generating components may comprise a thickener.

According to a fifth aspect of the invention, there is provided a method of increasing a duration of inhalation activity, the method comprising the steps of: providing the device as claimed in any one of the preceding claims; opening the barrier so that the foam-generating components react to generate the stable foam which flows to the outlet via the fluid flow path; and inhaling the stable foam with multiple inhalations.

According to a sixth aspect of the invention, there is provided a nicotine inhalation device for dispensing a stable foam to be inhaled by a user over a prolonged duration, the inhalation device comprising: a foam-generating-component receiving portion; a non-electrical inhalation cartridge having stable-foam-generating components for generating the stable foam, said components being receivable at or in the component receiving portion, at least one of said components being segregated from another of said components by an openable barrier; at least one of the stable-foam-generating components comprising nicotine; an outlet for dispensing the stable foam to the user; a fluid flow path which fluidly communicates the foam-generating-component receiving portion with the outlet so that when the barrier is opened the components interact to generate the stable foam which flows to the outlet via the fluid flow path to be inhaled by the user over a prolonged duration.

According to a seventh aspect of the invention, there is provided a cannabis-component inhalation device for dispensing a stable foam to be inhaled by a user over a prolonged duration, the inhalation device comprising: a foam-generating-component receiving portion; a non-electrical inhalation cartridge having stable-foam-generating components for generating the stable foam, said components being receivable at or in the component receiving portion, at least one of said components being segregated from another of said components by an openable barrier; at least one of the stable-foam-generating components comprising a cannabis component; an outlet for dispensing the stable foam to the user; a fluid flow path which fluidly communicates the foam-generating-component receiving portion with the outlet so that when the barrier is opened the components interact to generate the stable foam which flows to the outlet via the fluid flow path to be inhaled by the user over a prolonged duration.

According to an eighth aspect of the invention, there is provided a medicine and/or dietary supplement inhalation device for dispensing a stable foam to be inhaled by a user over a prolonged duration, the inhalation device comprising: a foam-generating-component receiving portion; a non-electrical inhalation cartridge having stable-foam-generating components for generating the stable foam, said components being receivable at or in the component receiving portion, at least one of said components being segregated from another of said components by an openable barrier; at least one of the stable-foam-generating components comprising a medicine and/or dietary supplement; an outlet for dispensing the stable foam to the user; a fluid flow path which fluidly communicates the foam-generating-component receiving portion with the outlet so that when the barrier is opened the components interact to generate the stable foam which flows to the outlet via the fluid flow path to be inhaled by the user over a prolonged duration.

According to a ninth aspect of the invention, there is provided a food or drink inhalation device for dispensing a stable foam to be inhaled by a user over a prolonged duration, the inhalation device comprising: a foam-generating-component receiving portion; a non-electrical inhalation cartridge having stable-foam-generating components for generating the stable foam, said components being receivable at or in the component receiving portion, at least one of said components being segregated from another of said components by an openable barrier; at least one of the stable-foam-generating components comprising a food or drink supplement; an outlet for dispensing the stable foam to the user; a fluid flow path which fluidly communicates the foam-generating-component receiving portion with the outlet so that when the barrier is opened the components interact to generate the stable foam which flows to the outlet via the fluid flow path to be inhaled by the user over a prolonged duration.

According to a tenth aspect of the invention, there is provided an inhalation apparatus for dispensing an inhalant to a user, the apparatus comprising: a component receiving container comprising a flexible material; components for generating an inhalant, said components being receivable at or in the component receiving container, at least one of said components being received within a further container from which said at least one component is dispensable by pressure applied to the further container via flexion; an outlet for dispensing the inhalant to the user; a fluid flow path which fluidly communicates the component receiving container with the outlet so that when said at least one component is dispensed from the further container the components interact to generate the inhalant which flows to the outlet via the fluid flow path to be inhaled by the user.

According to an eleventh aspect of the invention, there is provided a stable-foam inhalation device for dispensing a stable foam to be inhaled by a user over a prolonged duration, the inhalation device comprising: a cartridge receiving portion; a cartridge receivable in the cartridge receiving portion, the cartridge including at least one chamber for receiving a foam-generating-component and a perforable barrier fluidly communicable with said chamber; a protrusion at or adjacent to the cartridge receiving portion for perforating the barrier; an outlet for dispensing the stable foam to the user; a fluid flow path which fluidly communicates the cartridge receiving portion with the outlet so that when the barrier is perforated by the protrusion the chamber is fluidly communicated with the outlet.

According to a twelve aspect of the invention, there is provided a stable-foam inhalation-device cartridge comprising: a first chamber having an at least in part flexible wall, the first chamber containing a liquid foam-generating component; a second chamber having a further foam-generating component; an openable barrier between the first and second chamber; an outlet communicative with the second chamber for emitting stable foam therefrom; the flexible wall being manipulable to emit the liquid foam-generating component from the first chamber into the second chamber when the barrier is opened so that a stable foam is generated which is dispensable via the outlet.

According to a thirteenth aspect of the invention, there is provided a stable-foam inhalation-device cartridge comprising: a first chamber having an at least in part flexible wall, the first chamber containing a liquid foam-generating component; a second chamber containing a further foam-generating component; the liquid foam-generating component and the further foam-generating component being interactable to generate a stable foam; and at least one outlet associated with the cartridge for dispensing foam or at least one of the foam-generating components therefrom; the flexible wall being manipulable so that foam or the liquid foam-generating component is emitted from the outlet.

According to a fourteenth aspect of the invention, there is provided a liquid emission device comprising: a container with liquid received therein, the container including at least in part flexible wall for applying manual pressure to the liquid; the wall including an opening in the wall for emitting liquid from the container when manual pressure is applied to the liquid, or a perforable portion in which an opening can be formed for emitting liquid from the container when manual pressure is applied to the liquid.

According to a fifteenth aspect of the invention there is provided a stable-foam inhalation-device cartridge for a stable-foam inhalation device which dispenses a stable foam to be consumed by a user, the inhalation-device cartridge being devoid of electrical energisation means and comprises: a flexible mixing chamber having a first receiving portion for slidably receiving a first foam-generation element, and a second receiving portion for slidably receiving a second foam-generation element; the flexible mixing chamber having an access opening at a first end and being closed at a second end which is opposite the said first end, the access opening being dimensioned to receive the first and second foam-generation elements therethrough; an expansion chamber which is fluidly-communicable with the access opening of the flexible mixing chamber via a partitioning element, the partitioning element having one or more mixing members for agitating a consumable foam produced by the first and second foam-generation elements as it passes from the mixing chamber to the expansion chamber; and a discharge element which is at or adjacent to one end of the expansion chamber and which has an outlet opening for enabling a user to inhale the consumable foam from the expansion chamber, the discharge element including an inlet opening and a discharge conduit which interconnects the inlet opening with the outlet opening, the discharge conduit having a longitudinal extent which locates the inlet opening at a position closer to the partitioning element than to the outlet opening.

A stable foam, as opposed to only a short-lived effervescence and/or a rapidly propelled inhalant, may allow for a user to consume or inhale a substance contained within the foam slowly, over a prolonged duration and with multiple inhalations. This can provide a satisfying and pleasurable experience for the user. The cartridge being devoid of electrical energisation means reduces or eliminates a requirement for electrical charging. The flexible mixing chamber provides a convenient way of activating the foam-generation elements, for example in the instance that the foam-generation elements are manually activatable. The partitioning element spaces or separates the foam-generation elements from the outlet of the cartridge, which allows for the foam to expand into the space therebetween. Such a space is here defined by the expansion chamber. The mixing members or arms of the partitioning element define apertures through which the foam moves. The movement of the foam through the apertures may generate vortices which can help to mix the foam with any unreacted foam-generation elements to improve a consistency of the foam. The discharge element with a discharge conduit allows for foam to be inhaled away from an end of the cartridge. In other words, the foam may be drawn to the outlet from a more central region of the cartridge, which may improve the consistency of foam at the outlet.

Although the mixing chamber is described as being flexible, it will be appreciated that this may not be the case. For example, in the instance of non-manual activation means being considered. Additionally or alternatively, it could be envisaged that the mixing chamber may not have an access opening as such, for example if the mixing chamber and the expansion chamber were unitarily formed.

The second end of the mixing chamber may be integrally formed with the remainder of the mixing chamber, although it will be appreciated that a separable end cap may be used.

Whilst the partitioning element is described, a partitioning element may not be strictly necessary. Altematively, if a partitioning element is present, a mixing member may not necessarily required.

Although a discharge conduit is described for the discharge element, it will be appreciated that this may be omitted. Altematively, if the discharge conduit is present, it would be understood that the longitudinal extent of the discharge conduit might be such that the inlet opening is closer to the outlet opening than the partitioning element.

Although the cartridge is described as being devoid of electrical energisation means, it will be appreciated that this may be included. For example, the cartridge may include an electric motor for agitating a liquid to produce foam, and a battery for energising the electric motor.

Whilst the device is described as being for a stable foam, it will be appreciated that the device may be used with other inhalants or consumables.

Preferably, the flexible mixing chamber may comprise a thermoplastic elastomer. The thermoplastic elastomer can provide a food safe, durable and sufficiently flexible material for the mixing chamber.

Advantageously, the inlet opening may be off-centre relative to the expansion chamber. In a preferable embodiment, the inlet opening may be at or adjacent to a lateral wall of the expansion chamber. Such an arrangement allows for the inlet opening to be at an in use lower position. Therefore, as the foam is consumed and a level of foam reduces in the expansion chamber, a greater total amount of foam can be accessed since the remaining foam would occupy the lower portion of the expansion chamber under gravity.

Beneficially, the partitioning element may comprise a stop, the access opening and the expansion chamber being connectable to the partitioning element either side of the stop. This allows for convenient assembly of the cartridge, since the mixing chamber and the expansion chamber can be pushed over sealing surfaces of the partitioning element until the stop is reached.

In a preferable embodiment, the stop may be a ridge which extends around the perimeter of the partitioning element.

Preferably, the partitioning element comprises an axially extending protrusion which is extendable into the flexible mixing chamber for contacting the first or second foam-generation elements. This may provide a small area of contact on the first or second foam-generation elements which may assist with discharging a liquid therefrom, for example.

Optionally, a passageway through the partitioning element may widen from the flexible mixing chamber to the expansion chamber. The widening of the passageway may encourage foam to move from the mixing chamber to the expansion chamber.

Additionally, the passageway may be widened via a step. This may permit for convenient manufacture of the widening.

Advantageously, the inhalation-device cartridge may further comprise the first and second foam-generation elements.

Beneficially, the second foam-generation element may comprise a container of liquid. A liquid allows for dissolving of the first foam-generation element.

In a preferable embodiment, the liquid may be water. Water is an ingestible liquid and can be used with a wide variety of container materials. Other solvents may be considered.

Additionally, the liquid may be dispensable from the container via manual pressure applied to the container. As such, the user is able to conveniently dispense the liquid from the container without a tool.

Optionally, the container may be frangible via said manual pressure. A frangible, breakable, burstable or crushable container allows for the liquid to be dispensed from an otherwise sealed container.

Preferably, the container may include a hole in an exterior wall thereof through which the passage of liquid is prevented or limited when manual pressure is not applied to the container, and through which liquid is dispensable when manual pressure is applied to the container. This has the benefit that the container is not required to be broken to dispense liquid therefrom.

Advantageously, the container may be oriented so that the hole faces the first foam-generation element. The liquid is therefore dispensed directly onto the first foam-generation element, which may reduce or eliminate a requirement to shake the cartridge to better distribute the liquid, for example.

Preferably, the container may comprise a thermoplastic elastomer. The thermoplastic elastomer can provide a food safe, durable and sufficiently flexible material.

Advantageously, the first foam generating element comprises a carbonate and an acid. This allows for effervescence by producing carbon dioxide via a chemical reaction. A chemical reaction, as opposed to, for example, releasing a pressurised container, is more convenient for manufacturing purposes.

In a preferable embodiment, the carbonate comprises sodium bicarbonate and the acid comprises citric acid. These are two ingestible or food-safe components, although other carbonates and acids may be considered.

Beneficially, the first foam generating element comprises a stabiliser. A stabiliser allows for the conversion of an otherwise short-lived effervescence into a stable foam.

Optionally, the stabiliser comprises lectin and/or xanthan gum. Other stabilisers or thickeners may also be considered.

According to a sixteenth aspect of the invention there is provided a stable-foam inhalation device for dispensing a stable foam to be consumed by a user, the inhalation device comprising the cartridge as claimed in any one of the preceding claims and a carrier, the carrier including: a carrier body; a receiving portion for receiving the cartridge at or in the carrier body; a carrier inlet for fluid communication with the outlet of the discharge element when the carrier is received in or at the receiving portion; a carrier outlet for dispensing a stable foam to a user; and a conduit between the carrier inlet and the carrier outlet through the carrier body.

The use of a carrier and a cartridge allows for a disposable cartridge and a reusable carrier. As such, the convenience of a pre-loaded cartridge is provided, without requiring the entire device to be disposable. The carrier can therefore be designed to be large and ergonomic as well as including additional features, without great concern for material waste since the carrier is reusable.

It will be appreciated that the discharge conduit of the cartridge may extend through at least part of the holder. In fact, the discharge conduit could extend only through the holder and may not extend through the expansion chamber.

Preferably, the stable-foam inhalation device may further comprise a one-way valve in the conduit for permitting movement of foam from the carrier inlet to the carrier outlet and preventing or limiting movement of fluid from the carrier outlet to the carrier inlet. As such, the user can consume or inhale foam whilst being prevented or limited from exhaling into the device and disrupting a foam-distribution therein.

Advantageously, the one-way valve may be a duck-bill valve.

Beneficially, the stable-foam inhalation device may further comprise an indication element for indicating when foam is being inhaled. This indicates to the user and/or bystanders that the device is in use.

Additionally, the indication element may comprise a light-emitting device for lighting when foam is being inhaled. Lighting may be a less intrusive signal as compared to a sound, for example. It will be appreciated that the light-emitting device does not interfere with the main functionality of the inhalation device. In other words, the user can still consume foam whether or not the light is present and operational.

Preferably, the indication element may comprise a pressure sensor. This allows for the device to detect when the user is inhaling.

Optionally, the pressure sensor may be spaced apart from the conduit, the pressure sensor being in or at a sub-conduit which is fluidly communicable with the conduit. As such, the pressure sensor can be remote from the conduit outlet, which may be convenient for an electrical arrangement of the device.

Preferably, the carrier body defines the receiving portion, a part of the carrier body being moveable relative to a remainder of the carrier body for captively holding the cartridge. A moveable part or portion of the carrier body allows for the receiving portion to be changeable in size. In other words, said part can be moved to enlarge or elongate the receiving portion so the cartridge can be positioned therein. The part can then be moved back into position so as to secure the cartridge into position.

Advantageously, the stable-foam inhalation device may further comprise a biasing means for biasing said part of the carrier body towards the receiving portion. A biasing means permits for the part to be automatically moved back towards the cartridge and may secure the cartridge in place with a biasing force. This may be advantageous for users with reduced dexterity, for example users with arthritis.

Beneficially, the biasing means may be a spring.

Optionally, said part may be at or adjacent to the carrier inlet.

Preferably, the carrier body may include at least one side wall, the or each side wall including an access opening therein for providing visual or manual access to the cartridge. The access opening can, for example, permit for the cartridge to be directly engaged and moved. This may allow for the foam-generation elements to be manually manipulated through the mixing chamber walls and activated, for example. Furthermore, permitting movement of the cartridge through the access openings may assist with releasing the cartridge from the receiving portion. Alternatively or additionally, the access opening may allow for a visual inspection by the user of the production or level of foam remaining in the cartridge. The use of an access opening permits for access to the cartridge, whilst allowing for the side wall to extend further up the side of the cartridge to more securely laterally hold the cartridge in position. The access opening can also allow for leverage to be applied underneath the cartridge to assist with removal from the receiving portion.

Beneficially, the carrier body may have two side walls, each side wall having an access opening therein. Access openings in each side wall can allow for the cartridge to be gripped between fingers.

Advantageously, the carrier body and the or each access opening may be elongate, a longitudinal extent of the or each access opening being aligned with that of the carrier body. This may allow for a greater proportion of the cartridge to be visible.

Additionally, the access opening may extend towards the biasing means. This can allow for the cartridge to be conveniently moved towards the biasing means.

In a preferable embodiment, the or each access opening may be tapered.

According to a seventeenth aspect of the invention there is provided a nicotine-stable-foam inhalation device for dispensing a stable foam which comprises nicotine to be consumed by a user, the device comprising: a stable-foam inhalation-device cartridge which is devoid of electrical energisation means and includes a flexible mixing chamber having a first receiving portion for slidably receiving a first foam-generation element, and a second receiving portion for slidably receiving a second foam-generation element, the flexible mixing chamber having an access opening at a first end and being closed at a second end which is opposite the said first end, the access opening being dimensioned to receive the first and second foam-generation elements therethrough, an expansion chamber which is fluidly-communicable with the access opening of the flexible mixing chamber via a partitioning element, the partitioning element having one or more mixing members for agitating a consumable foam produced by the first and second foam-generation elements as it passes from the mixing chamber to the expansion chamber, a discharge element which is at or adjacent to one end of the expansion chamber and which has an outlet opening for enabling a user to inhale the consumable foam from the expansion chamber, the discharge element including an inlet opening and a discharge conduit which interconnects the inlet opening with the outlet opening, the discharge conduit having a longitudinal extent which locates the inlet opening at a position closer to the partitioning element than to the outlet opening; first and second foam-generation elements received in the first receiving portion and second receiving portion respectively, the first and/or second foam-generation element comprising nicotine; and a carrier which includes a carrier body; a receiving portion which receives the cartridge at or in the carrier body; a carrier inlet in fluid communication with the outlet of the discharge element; and a carrier outlet for dispensing the foam to the user.

According to a eighteenth aspect of the invention there is provided a method of consuming nicotine, the method comprising: a) assembling the nicotine-stable-foam inhalation device according to the seventeenth aspect of the invention by receiving the cartridge at the receiving portion of the carrier; b) actuating the first and/or second foam-generation elements so that the first and second foam-generation elements together produce a stable foam which comprises nicotine; and c) inhaling said stable foam from the carrier outlet.

According to a nineteenth aspect of the invention there is provided a cannabis-component-stable-foam inhalation device for dispensing a stable foam which comprises a component of cannabis to be consumed by a user, the device comprising: a stable-foam inhalation-device cartridge which is devoid of electrical energisation means and includes a flexible mixing chamber having a first receiving portion for slidably receiving a first foam-generation element, and a second receiving portion for slidably receiving a second foam-generation element, the flexible mixing chamber having an access opening at a first end and being closed at a second end which is opposite the said first end, the access opening being dimensioned to receive the first and second foam-generation elements therethrough, an expansion chamber which is fluidly-communicable with the access opening of the flexible mixing chamber via a partitioning element, the partitioning element having one or more mixing members for agitating a consumable foam produced by the first and second foam-generation elements as it passes from the mixing chamber to the expansion chamber, a discharge element which is at or adjacent to one end of the expansion chamber and which has an outlet opening for enabling a user to inhale the consumable foam from the expansion chamber, the discharge element including an inlet opening and a discharge conduit which interconnects the inlet opening with the outlet opening, the discharge conduit having a longitudinal extent which locates the inlet opening at a position closer to the partitioning element than to the outlet opening; first and second foam-generation elements received in the first receiving portion and second receiving portion respectively, the first and/or second foam-generation element comprising a cannabis component; and a carrier which includes a carrier body; a receiving portion which receives the cartridge at or in the carrier body; a carrier inlet in fluid communication with the outlet of the discharge element; and a carrier outlet for dispensing the foam to the user.

According to a twentieth aspect of the invention there is provided a method of consuming a cannabis component, the method comprising: a) assembling the cannabis-component-stable-foam inhalation device according to a nineteenth aspect of the invention by receiving the cartridge at the receiving portion of the carrier; b) actuating the first and/or second foam-generation elements so that the first and second foam-generation elements together produce a stable foam which comprises a component of cannabis; and c) inhaling said stable foam from the carrier outlet.

According to a twenty-first aspect of the invention there is provided a medicine and/or dietary supplement stable-foam inhalation device for dispensing a stable foam which comprises a medicine and/or dietary supplement to be consumed by a user, the device comprising: a stable-foam inhalation-device cartridge which is devoid of electrical energisation means and includes a flexible mixing chamber having a first receiving portion for slidably receiving a first foam-generation element, and a second receiving portion for slidably receiving a second foam-generation element, the flexible mixing chamber having an access opening at a first end and being closed at a second end which is opposite the said first end, the access opening being dimensioned to receive the first and second foam-generation elements therethrough, an expansion chamber which is fluidly-communicable with the access opening of the flexible mixing chamber via a partitioning element, the partitioning element having one or more mixing members for agitating a consumable foam produced by the first and second foam-generation elements as it passes from the mixing chamber to the expansion chamber, a discharge element which is at or adjacent to one end of the expansion chamber and which has an outlet opening for enabling a user to inhale the consumable foam from the expansion chamber, the discharge element including an inlet opening and a discharge conduit which interconnects the inlet opening with the outlet opening, the discharge conduit having a longitudinal extent which locates the inlet opening at a position closer to the partitioning element than to the outlet opening; first and second foam-generation elements received in the first receiving portion and second receiving portion respectively, the first and/or second foam-generation element comprising a medicine and/or dietary supplement; and a carrier which includes a carrier body; a receiving portion which receives the cartridge at or in the carrier body; a carrier inlet in fluid communication with the outlet of the discharge element; and a carrier outlet for dispensing the foam to the user.

According to an twenty-second aspect of the invention there is provided a method of consuming a medicine and/or dietary supplement, the method comprising: a) assembling the medicine and/or dietary supplement stable-foam inhalation device according to a twenty-first aspect of the invention by receiving the cartridge at the receiving portion of the carrier; b) actuating the first and second foam-generation elements so that the first and second foam-generation elements together produce a stable foam which comprises a medicine and/or dietary supplement; and c) inhaling said stable foam from the carrier outlet.

According to a twenty-third aspect of the invention there is provided a food or drink stable-foam inhalation device for dispensing a stable foam which comprises a food or drink substance to be consumed by a user, the device comprising: a stable-foam inhalation-device cartridge which is devoid of electrical energisation means and includes a flexible mixing chamber having a first receiving portion for slidably receiving a first foam-generation element, and a second receiving portion for slidably receiving a second foam-generation element, the flexible mixing chamber having an access opening at a first end and being closed at a second end which is opposite the said first end, the access opening being dimensioned to receive the first and second foam-generation elements therethrough, an expansion chamber which is fluidly-communicable with the access opening of the flexible mixing chamber via a partitioning element, the partitioning element having one or more mixing members for agitating a consumable foam produced by the first and second foam-generation elements as it passes from the mixing chamber to the expansion chamber, a discharge element which is at or adjacent to one end of the expansion chamber and which has an outlet opening for enabling a user to inhale the consumable foam from the expansion chamber, the discharge element including an inlet opening and a discharge conduit which interconnects the inlet opening with the outlet opening, the discharge conduit having a longitudinal extent which locates the inlet opening at a position closer to the partitioning element than to the outlet opening; first and second foam-generation elements received in the first receiving portion and second receiving portion respectively, the first and/or second foam-generation element comprising a food or drink substance; and a carrier which includes a carrier body; a receiving portion which receives the cartridge at or in the carrier body; a carrier inlet in fluid communication with the outlet of the discharge element; and a carrier outlet for dispensing the foam to the user.

According to a twenty-fourth aspect of the invention there is provided a method of consuming a food or drink substance, the method comprising: a) assembling the food or drink stable-foam inhalation device according to a twenty-third aspect of the invention by receiving the cartridge at the receiving portion of the carrier; b) actuating the first and second foam-generation elements so that the first and second foam-generation elements together produce a stable foam which comprises a food or drink substance; and c) inhaling said stable foam from the carrier outlet.

According to an twenty-fifth aspect of the invention there is provided a non-electrical inhalation cartridge for a stable-foam inhalation device which dispenses a stable foam to be inhaled by a user over a prolonged duration, the inhalation device comprising: a foam-generating-component receiving portion; stable-foam-generating components for generating the stable foam, said components being receivable at or in the component receiving portion, at least one of said components being segregated from another of said components by an openable barrier; an outlet for dispensing the stable foam to the user; a conduit which fluidly communicates the foam-generating-component receiving portion with the outlet so that when the barrier is opened the components interact to generate the stable foam which flows to the outlet via the conduit to be inhaled by the user over a prolonged duration.

According to a twenty-sixth aspect of the invention there is provided a method of increasing a duration of inhalation activity, the method comprising the steps of: a) providing the cartridge according to the twenty-fifth aspect of the invention; b) opening the barrier so that the foam-generating components react to generate the stable foam which flows to the outlet via the conduit; and c) inhaling the stable foam with multiple inhalations.

According to a twenty-seventh aspect of the invention there is provided a inhalation apparatus for dispensing an inhalant to a user, the apparatus comprising: a component receiving container comprising a flexible material; components for generating an inhalant, said components being receivable at or in the component receiving container, at least one of said components being received within a sub-container from which said at least one component is dispensable by pressure applied to the sub-container via flexion of the component receiving container; an outlet for dispensing the inhalant to the user; a conduit which fluidly communicates the component receiving container with the outlet so that when said at least one component is dispensed from the sub-container the components interact to generate the inhalant which flows to the outlet via the conduit to be inhaled by the user.

The invention will now be more particularly described, by way of example only, with reference to the accompanying drawings, in which:
Figure 1 shows a perspective view of a first embodiment of a carrier of a first embodiment of a foam inhalation device in accordance with a first aspect of the invention;
Figure 2 shows a side cutaway view of the carrier of Figure 1;
Figure 3 shows a bottom cutaway view of the carrier of Figure 1;
Figure 4 shows a perspective view of a first embodiment of a cartridge of the first embodiment of the foam inhalation device in accordance with the first aspect of the invention, for receipt by the carrier of Figure 1;
Figure 5 shows a side view of the cartridge of Figure 4;
Figure 6 shows an end view of the cartridge of Figure 4;
Figure 7 shows a top view of a second embodiment of a stable-foam inhalation-device cartridge according to thirteenth and twenty-fifth aspects of the invention;
Figure 8 shows a side view of the stable-foam inhalation-device cartridge of Figure 7;
Figure 9 shows a perspective view of the stable-foam inhalation-device cartridge of Figure 8;
Figure 10 shows a perspective view of a partitioning element of the stable-foam inhalation-device cartridge of Figure 7;
Figure 11 shows a perspective view of a second foam-generation element of the stable-foam inhalation-device cartridge of Figure 7;
Figure 12 shows a perspective view of a second embodiment of a stable-foam inhalation device according to fourth and sixteenth aspects of the invention, with the stable-foam inhalation-device cartridge of Figure 7 therein;
Figure 13 shows a side cut away view of the stable-foam inhalation device of Figure 12 without the stable-foam inhalation-device cartridge;
Figure 14 shows an enlarged view of an inlet of the stable-foam inhalation device of Figure 12 without the stable-foam inhalation-device cartridge;
Figure 15 shows an exploded view of a rear part of the stable-foam inhalation device of Figure 12;
Figure 16 shows a perspective view of the assembled rear part of Figure 15;
Figure 17 shows a top view of a third embodiment of a carrier of a third embodiment of a stable-foam inhalation device in accordance with fourth and sixteenth aspects of the invention, a block arrow showing a direction of insertion of a cartridge;
Figure 18 shows a perspective view of the carrier of Figure 17;
Figure 19 shows a side view of the carrier of Figure 17;
Figure 20 shows a perspective view of a fourth embodiment of a stable-foam inhalation device in accordance with fourth and eleventh aspects of the invention.
Figure 21 shows a perspective view of a fourth embodiment of a stable-foam inhalation-device cartridge of the stable-foam inhalation device of Figure 20; and
Figure 22 shows a side view of the stable-foam inhalation-device cartridge of Figure 21; and
Figure 23 shows a side cut-away view of the stable-foam inhalation device of Figure 20.

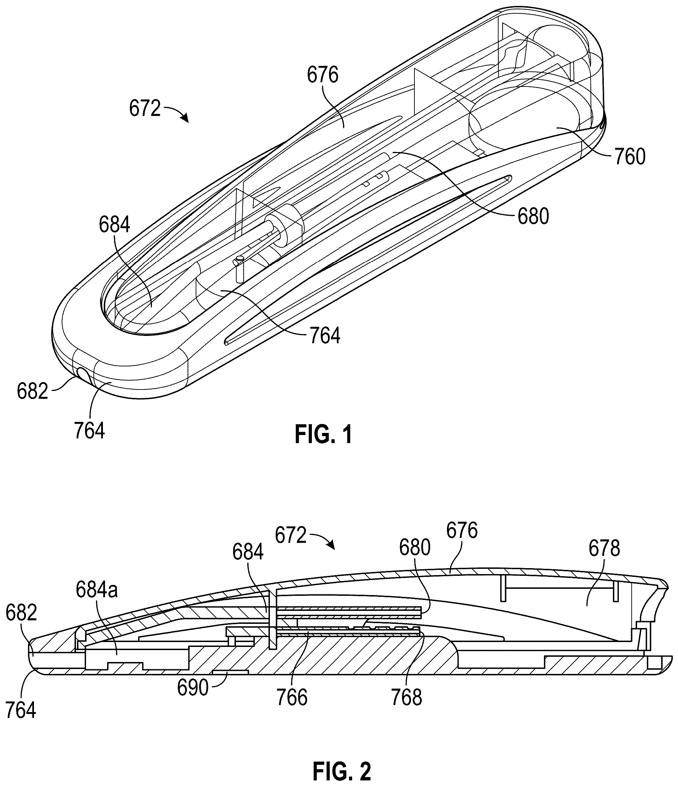

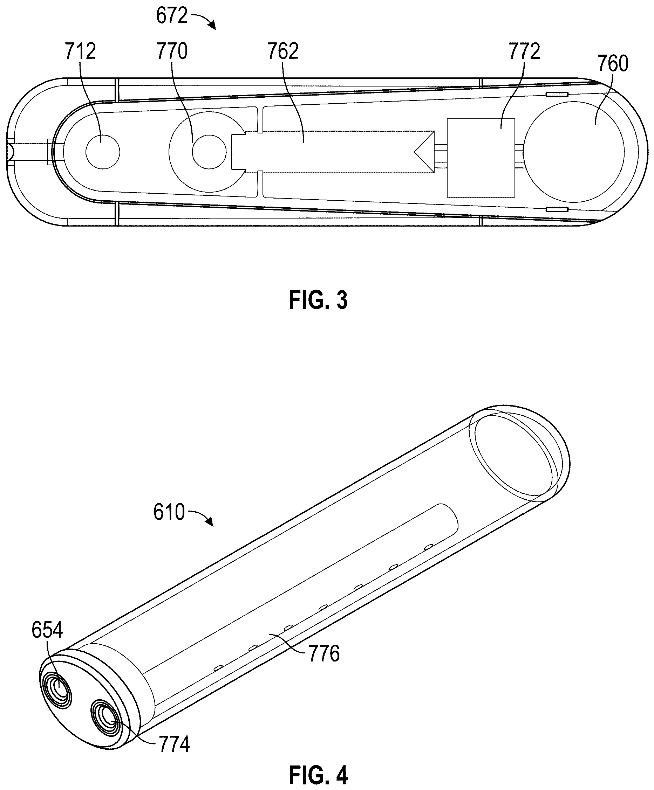

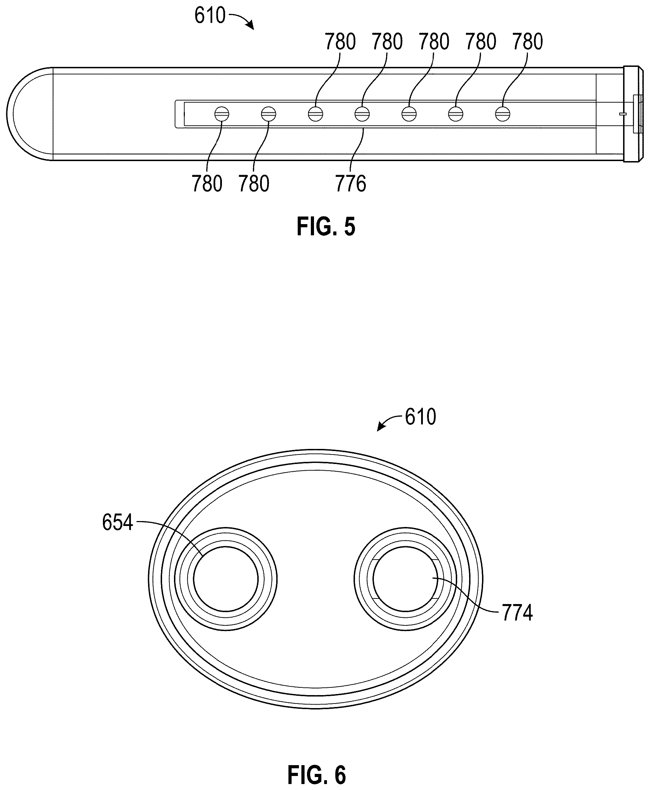

Referring firstly to Figures 1 to 6, a foam inhalation device or a foam drawing device comprises a carrier 672, as shown in Figures 1 to 3, with a cartridge 610, as shown in Figures 4 to 6, received therein. The device is electrically operated and here is powered by a battery 760 and so is battery-operated, although it will be appreciated that other electrical power sources may be considered.

Foam is generated by agitating a liquid with an agitation means, and the agitation means comprises at least an electric motor 762 and the battery 760. The agitation means may otherwise be referred to as an agitator.

Referring now in particular to Figures 1 to 3, the carrier 672 has a carrier body 676 which preferably houses the motor 762 and the battery 760. The carrier 672 further includes a receiving portion 678 for receiving the cartridge 610 at or in the carrier body 676. Here the receiving portion 678 is a recess or open-ended chamber defined by the body 676 and sized to receive at least part of the cartridge 610 therein. The receiving portion 678 preferably only receives part of the cartridge 610 therein, with an end of the cartridge 610 protruding from the end of the receiving portion 678.

The carrier 672 further includes a carrier foam inlet 680 for receiving foam from the cartridge 610 when the cartridge 610 is received in the receiving portion 678. The carrier 672 also includes a carrier foam outlet 682, which is here defined by a mouthpiece 764, for dispensing the foam to a user. A carrier foam conduit 684, passageway or flow path connects the carrier foam inlet 680 with the carrier foam outlet 682.

At least an end portion of the carrier 672 foam conduit adjacent to the carrier foam inlet 680 preferably projects or protrudes into the receiving portion 678 so as to permit the carrier foam conduit 684 to perforate the cartridge 610. However, it will be appreciated that this may not be necessary and the conduit may not project or protrude and may instead abuttingly connect with the cartridge 610.

The carrier foam conduit 684 may include a chamber 684a proximal to the mouthpiece 764. However, it will be appreciated that this may be omitted.

A one-way valve may be in the carrier foam conduit 684 for permitting movement of foam from the carrier foam inlet 680 to the carrier foam outlet 682 and preventing or limiting movement of fluid from the carrier foam outlet 682 to the carrier foam inlet 680.

The carrier further comprises a carrier air inlet 690 and an air-flow conduit 766 which extends from the carrier air inlet 690 and terminates in a carrier air outlet 768 at, in or adjacent to the receiving portion 678. The carrier air inlet 690 is open to the exterior of the carrier 672 in use and is separate to and preferably away from the carrier foam outlet 682. For example, the carrier foam outlet 682 is preferably positioned so as not be covered by a user's mouth in use when a user is drawing at the carrier foam outlet 682. Preferably the carrier air inlet 690 is in a base of the carrier 672. Similar to the carrier foam conduit 684, the air-flow conduit 766 preferably projects or protrudes into the receiving portion 678 so as to permit perforation of the cartridge 610.

The air-flow conduit 766 and the carrier air outlet 768 are in use below or beneath the carrier foam conduit 684 and the carrier foam inlet 680.

The agitation means preferably comprises an air moving means which is driven by the electric motor 762 and is for moving air from the carrier air inlet 690 to the carrier air outlet 768. The air moving means may be an air pump 770. For example, the air pump 770 may be a fan or impeller rotatable by the electric motor 762, although other forms of air pump 770 may be considered.

The device preferably includes a pressure sensor 712, which is here housed in the carrier 672. The pressure sensor 712 is fluidly communicated with the carrier foam outlet 682 so as to detect when negative pressure is applied to the carrier foam outlet 682. Here the pressure sensor 712 is positioned at, adjacent to or in the chamber 684a which is proximal to the mouthpiece 764.

The device includes a processor, which is here provided on or by a printed circuit board 772, which is again housed in the carrier 672. The battery 760, the motor 762 and the pressure sensor 712 are connected to the printed circuit board 772 so that when the pressure sensor 712 detects negative pressure the processor activates the motor 762 by providing power from the battery 760 which drive the air pump 770.

The electric motor 762 is preferably a brushless electric motor.

The battery 760 may be rechargeable, and as such there may be a power terminal to permit recharging. Additionally or alternatively, the battery 760 may be removable from the carrier 672 to permit replacement. The battery 760 may be neither replaceable or rechargeable, and so the carrier 672 may be required to be disposed after discharge of the battery 760.

Referring now in particular to Figures 4 to 6, the cartridge 610 has a cartridge foam outlet 654 and defines a container for containing a liquid for producing the foam via agitation.

The liquid may comprise at least one foaming agent, surfactant, stabiliser and/or thickener to permit generation of foam via agitation. This may include lectin and/or xanthan gum. The foaming agent, and liquid as a whole, should be food-safe to permit consumption by a person.

The liquid may include an acid and/or a carbonate, such as citric acid and/or sodium bicarbonate. In the case that an acid and carbonate were included in the cartridge, they may have already produced a foam and/or carbon dioxide and there may be the resulting salt, such as sodium citrate, in the cartridge 610 before use of the device. If the carbon dioxide from such a reaction is still being produced whilst the cartridge 610 is sealed, then this may spontaneously produce some foam upon opening of the cartridge 610 due to a reduction in pressure resulting in release of dissolved carbon dioxide. However, even in this case, the primary foam generation method is via agitation.

The cartridge 610 is preferably elongate and tubular or substantially tubular. Here the cartridge 610 has a flat end, which includes the cartridge foam outlet 654, and a rounded end opposing the flat end.

The cartridge 610 may comprise a flexible material to allow manual manipulation thereof, although it will be appreciated that this may not be the case and the cartridge 610 may comprise of a rigid material.

The cartridge 610 preferably also comprises a cartridge air inlet 774. The cartridge air inlet 774 is preferably at or adjacent to the cartridge foam outlet 654, for example being in the flat end, although it will be appreciated that it may be positioned elsewhere. The cartridge air inlet 774 is preferably in use below the cartridge foam outlet 654.

The cartridge 610 preferably has an air-injection conduit 776 which extends from the cartridge air inlet 774 into an interior 778 of the cartridge 610 for injecting air into the cartridge 610 via at least one opening 780 in the air-injection conduit 776. Preferably, the air-injection conduit 776 is elongate and has a plurality of openings 780 therein which are spaced apart from each other in a longitudinal extent of the air-injection conduit 776. Preferably, there is no opening in an end of the air-injection conduit 776.

There may be at least one opening 780 on two opposing sides of the air-injection conduit 776. Preferably, the openings 780 on each side are aligned with each other.

Each opening 780 may be circular in shape. Preferably, there are at least five openings 780, here there being seven openings 780 on each opposing side of the air-injection conduit 776.

Alternatively, each opening 780 may have the shape of a segment of a circle and/or may be semi-circular. In this case, each side of the air-injection conduit 776 may have multiple pairs of openings 780, each opening 780 of a pair is spaced apart from each other in a lateral direction of the conduit. Preferably, there are seven pairs of openings 780 at each side of the conduit.

The cartridge air inlet 774 and the cartridge foam outlet 654 are each closed by a perforable or otherwise openable seal. For example, here each of the cartridge air inlet 774 and the cartridge foam outlet 654 has a perforable seal which is preferably also flexible, such as a silicone seal.

The carrier foam conduit 684 and the air-flow conduit 766 of the carrier 672 are arranged to perforate the cartridge foam outlet 654 and the cartridge air inlet 774 respectively, when the cartridge 610 is received in the receiving portion 678 of the carrier 672. This is achieved by the projection of the conduits into the receiving portion 678, as previously described. Additionally, a width or diameter of each conduit is sized so as to be received within the respective outlet and inlet. A border portion of the flexible seals may form a seal with the respective conduits so that leaks of liquid or foam is reduced. Additionally or alternatively, the conduits may be sized so as to sealingly engage with a perimeter of the outlet or inlet.

The cartridge 610 and the carrier 672 are configured so that the cartridge air inlet 774 is connected with the carrier air outlet 768, and the cartridge foam outlet 654 is connected with the carrier foam inlet 680, when the cartridge 610 is received in the receiving portion 678. Although not shown, to reduce the risk of misconnection between the carrier air outlet 768 and the cartridge foam outlet 654 and the cartridge air inlet 774 and the carrier foam inlet 680, the cartridge 610 and/or receiving portion 678 may be shaped to only permit receipt of the cartridge 610 in a correct orientation. For example, the cartridge 610 may have a protrusion and the carrier 672 may have a recess or groove so that the cartridge 610 and carrier 672 can only be correctly relatively positioned. Alternatively, the inlets and outlets may be non-symmetrically positioned so as to permit connection in only one orientation.

The device may be arranged to only operate the agitation means when a cartridge 610 is received in the carrier 672. To achieve this, the carrier 672 is preferably configured to detect when a cartridge 610 is received in the receiving portion 678. The carrier 672 may have a switch which is operated by the cartridge 610 being received in the receiving portion 678. For example, the carrier 672 may have exposed electrical contacts or terminals, with the cartridge 610 having an electrically conducting portion which is positioned on the cartridge 610 so that, when the cartridge 610 is received in the receiving portion 678, the electrical contacts are connected by the electrically conducting portion. Altematively, the carrier 672 may have a manual or pressure sensitive switch or button which is depressed when the cartridge 610 is received in the receiving portion 678. Either switch may be connected with the processor on the printed circuit board 772, so that when the cartridge 610 is received in the carrier 672 the processor is aware that the cartridge 610 is present. The processor may therefore only operate the agitation means when the cartridge 610 is detected and when suction is detected on the mouthpiece 764 by the pressure or airflow-sensor. This is to prevent unintentional operation of the motor and thus prevent draining of the battery 760.

In use, when a user wishes to consume foam, the cartridge 610, pre-loaded with the liquid, is inserted into the receiving portion 678. The carrier foam conduit 684 and the air-flow conduit 766 perforates or penetrates the cartridge foam outlet 654 and the cartridge air inlet 774 respectively. The cartridge 610 operates the switch so that the processor detects the presence of the cartridge 610.

The user then draws, inhales or sucks on the mouthpiece 764 of the carrier 672. This creates low pressure in the carrier foam conduit 684 which is detected by the pressure sensor 712. Since the processor is aware of the presence of the cartridge 610, the processor activates the motor which draws power from the battery 760 to operate the air pump 770. The air pump 770 draws air from the carrier air inlet 690, along the air-flow conduit 766 and into the cartridge 610 via the carrier air outlet 768. Air is injected into the liquid via the openings 780 in the air-injection conduit 776. As such, the liquid is effectively bubbled with air. The liquid is transformed into foam via this agitation.

The suction applied by the user causes the foam to be drawn out of the cartridge 610 via the cartridge foam outlet 654 and along the carrier foam conduit 684. The user thereby draws foam into their mouth via the carrier foam outlet 682. The user may stop inhaling once sufficient foam has been drawn, which may deactivate the agitation means. Draws on the mouthpiece 764 to generate foam via agitation may be repeated to permit the user to consume more foam until the liquid in the cartridge 610 has been consumed.

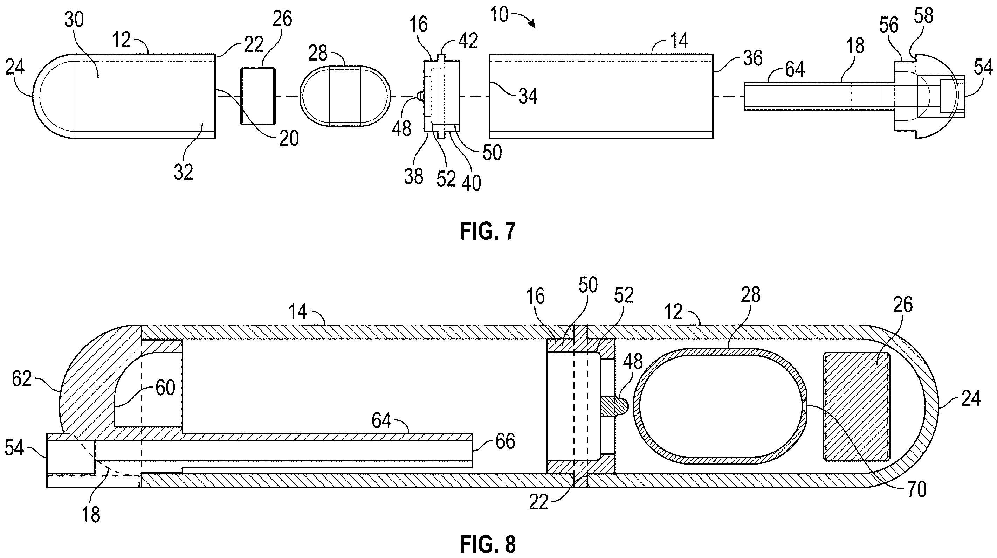

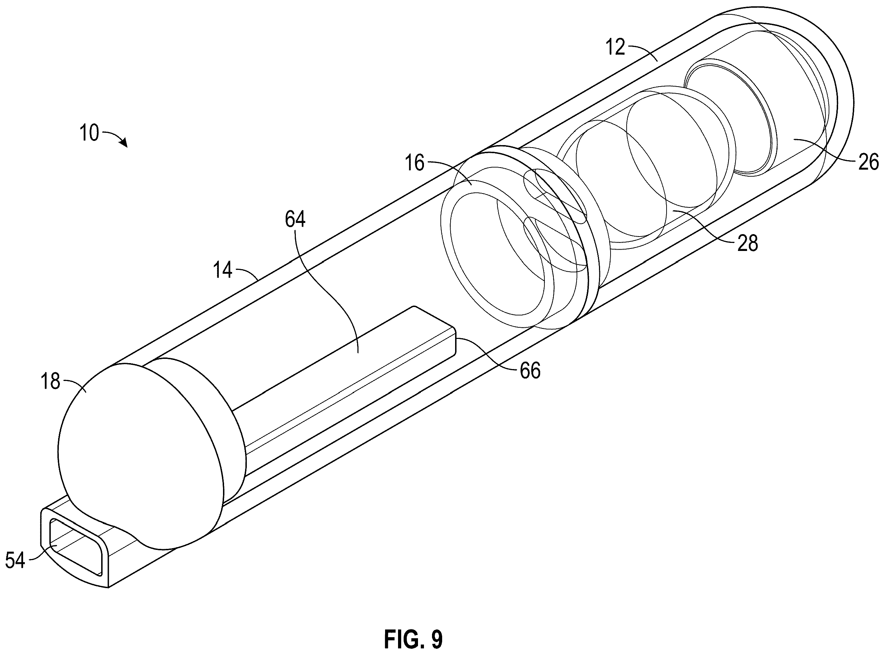

Referring now to Figures 7 to 9 there is shown a second embodiment of a stable-foam inhalation-device cartridge 10. The second embodiment of the inhalation-device cartridge 10 comprises a flexible mixing chamber 12, an expansion chamber 14, a partitioning element 16, and a discharge element 18.

The flexible mixing chamber 12 has an access opening 20 at a first end 22 and is closed at a second end 24 which is opposite the first end 22. The access opening 20 is dimensioned to receive first and second foam-generation elements 26, 28 therethrough. The access opening 20 is circular or substantially circular in shape, although it will be appreciated that other shapes may be considered such as square or triangular. Similarly, the flexible mixing chamber 12 has a circular cross-section, although other shapes may be considered. Preferably, the second end 24 is curved such that an end portion associated with the second end 24 may be considered to be semi-spherical. However, this may not be necessary, and the second end 24 could be planar or flat.

The flexible mixing chamber 12 has a first receiving portion 30 for slidably receiving the first foam-generation element 26, and a second receiving portion 32 for slidably receiving the second foam-generation element 28. The first receiving portion 30 is distal to the access opening 20 as compared to the second receiving portion 32 and is here at or adjacent to the second end 24. The second receiving portion 32 is at or adjacent to the access opening 20. The flexible mixing chamber 12 is preferably sized so that the first receiving portion 30 and the second receiving portion 32 together are defined across a majority of the length of the flexible mixing chamber 12, and more preferable are defined across substantially the entire length of the flexible mixing chamber 12.

The flexible mixing chamber 12 preferably comprises a thermoplastic elastomer which provides the desired flexibility of the mixing chamber 12. However, it will be appreciated that other flexible materials may be considered such as silicone, other elastomeric materials, for example rubber, or other flexible plastics. The flexibility of the mixing chamber 12 allows for compressive manual actuation or activation of the first and/or second foam-generation elements. However, in the instance of other actuation or activation methods, the mixing chamber 12 may not necessarily be flexible. The flexible mixing chamber 12 may preferably be transparent or translucent.

The expansion chamber 14 is preferably cylindrical or substantially cylindrical and may be considered to be a tube. However, other shapes may be considered. For example, the expansion tube may have a cross-sectional shape of that of a flattened circle. In other words, the expansion tube may have an elliptical cross-section. Any other shape may be considered, for example rectangular or triangular. This may similarly apply to the mixing chamber 12, partitioning element 16 and discharge element 18. The expansion chamber 14 preferably has a mixing-chamber proximal opening 34 and a discharge-element proximal opening 36. Here each opening is identical, although it will be appreciated that this may not necessarily be the case. The expansion chamber 14 is also formed from a flexible material, such as a thermoplastic elastomer, although it will be appreciated that this may not be necessary. The expansion chamber 14 may preferably be transparent or translucent.

The mixing-chamber proximal opening 34 of the expansion chamber 14 is fluidly communicable with the access opening 20 of the flexible mixing chamber 12 via the partitioning element 16. The partitioning element 16 preferably sealingly connects the expansion chamber 14 with the flexible mixing chamber 12. In other words, the partitioning element 16 connects and seals the expansion chamber 14 and the mixing chamber 12 together to prevent leakage of foam.

To achieve the sealing connection, the partitioning element 16 has mixing-chamber sealing surface 38 and an expansion-chamber sealing surface 40. Preferably each sealing surface 38, 40 is annular or substantially annular. Diameters of the mixing-chamber sealing surface 38 and an expansion-chamber sealing surface 40 are similar or identical to the diameters of the access opening 20 and the mixing-chamber proximal opening 34 of the expansion chamber 14 respectively. This is such that the sealing surfaces 38, 40 form an interference fit with the relevant opening and/or end portion of the expansion chamber 14 or mixing chamber 12. This prevents or limits leakage of foam or other components from either chamber. The sealing surfaces 38, 40 are receivable within the respective chamber to abut the inside wall thereof. The access opening 20 and the mixing-chamber proximal opening 34 preferably have similar or identical diameters and as such it will be appreciated that either sealing surface could feasibly be used for either opening. However, this may not be the case and the diameters of the access opening 20 and the opening of the expansion chamber 14 may be different.

The partitioning element 16 preferably comprises an exterior stop 42, the mixing chamber 12 and the expansion chamber 14 being connectable to the partitioning element 16 either side of the stop 42. The stop 42 is here a ridge which extends annularly around a perimeter of the partitioning element 16 and separates the two sealing surfaces 38, 40 from each other.

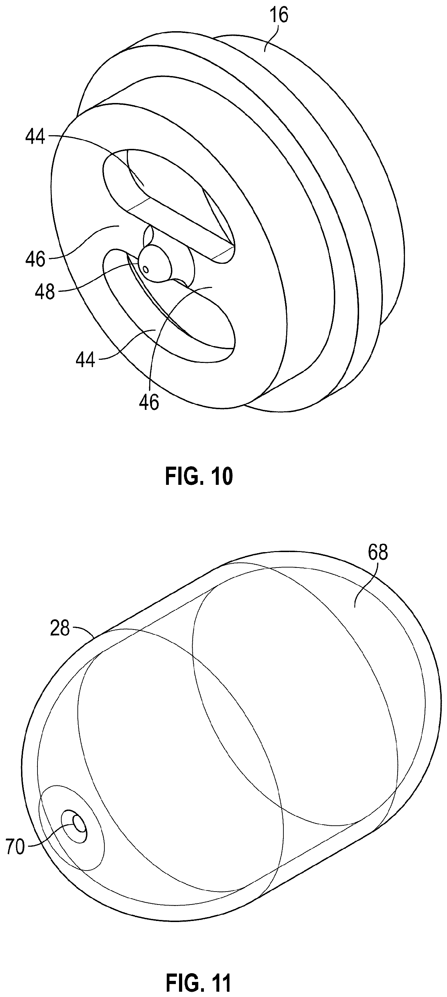

Referring now to Figure 10, the partitioning element 16 further comprises at least one hole 44 or through-bore for fluidly communicating the expansion chamber 14 with the access opening 20. Preferably, there are two separate holes 44, the holes 44 separated by at least one, and here two, mixing members 46. The mixing members 46 may be considered to be arms. Additionally, the mixing members 46 may prevent or limit the movement of the foam-generation elements in their initial form from the mixing chamber 12 to the expansion chamber 14. The holes 44 are preferably curved and/or the openings of the holes 44 are elongate such that the holes 44 may be considered to be slots.

The partitioning element 16 may additionally include a protrusion 48 which may extend axially into the mixing chamber 12. One protrusion 48 is shown, although it will be anticipated that multiple protrusions 48 may be considered. Additionally, the protrusion 48 is shown to be central on the partitioning element 16, although it will be appreciated that it may be off-centre. The protrusion is preferably tapered or pointed. In some instances a side wall of the mixing chamber could also include a protrusion.

Referring again to Figures 7 and 8 a passageway 50, at least in part defined by the holes 44, through the partitioning element 16 widens from the mixing-chamber sealing surface 38 to the expansion-chamber sealing surface 40. This widening of the passageway 50 is preferably abrupt, for example being formed by a step 52.

The discharge element 18 is at or adjacent to one end of the expansion chamber 14 and has an outlet opening 54 for dispensing or discharging foam therefrom. The discharge element 18 here closes the discharge-element proximal opening 36 at said end of the expansion chamber 14. As such, the discharge element 18 includes a sealing surface 56 which connects with and seals the expansion chamber 14 in the same or a similar way as the sealing surfaces of the partitioning element 16. The sealing surface 56 of the discharge element 18 is therefore annular, has a diameter which is similar or identical to that of the proximal opening of the expansion chamber 14 and is receivable therein. The discharge element 18 further includes a stop 58 to prevent over-insertion of the discharge element 18 into the expansion chamber 14.

An interior surface 60 of the discharge element 18 is concave or substantially concave which may assist with redirecting foam. An exterior surface 62 of the discharge element 18 is convex.

The outlet opening 54 preferably protrudes and/or is separated from the exterior surface of a body of the discharge element 18. The outlet opening 54 is off-centre relative to the remainder of the discharge element 18, being at or adjacent to an in use lower portion of the discharge element 18.

The discharge element 18 further includes a discharge conduit 64 and an inlet opening 66, the discharge conduit 64 interconnects the inlet opening 66 and the outlet opening 54 so that they are in fluid communication. The discharge conduit 64 has a longitudinal extent which locates the inlet opening 66 at a position closer to the partitioning element 16 than to the outlet opening 54. Additionally, the inlet opening 66 is located off-centre relative to the remainder of the discharge element 18 and/or the expansion chamber 14. In use, the inlet opening 66 is preferably at or adjacent to a lower interior surface of the expansion chamber 14.

The flexible mixing chamber 12 includes first and second foam-generation elements 26, 28. These are positioned inside the mixing chamber 12 in the corresponding receiving portions 30, 32. With the foam-generation elements 26, 28 received therein, the partitioning element 16 is connected to the mixing chamber 12, the expansion chamber 14 is connected to the partitioning element 16 and the discharge element 18 is connected to the expansion chamber 14. These parts may be adhered together to prevent disassembly or separation. Additionally, it will be appreciated that two or more of the parts may be unitarily formed together.

When the substances which comprise the first and second foam-generation elements 26, 28 are reacted or interacted together they produce a stable foam. This is preferably achieved by effervescence which is stabilised and/or thickened into a foam.

For example, the first foam-generation element 26 comprises a carbonate, for example sodium bicarbonate, and an acid, for example citric acid. Although sodium bicarbonate and citric acid are preferred, it will be appreciated that any other ingestible or food safe carbonate, for example calcium carbonate, or acid, for example tartaric acid, may be considered. The carbonate and the acid are here in solid form, for example being powdered and compressed into the first foam-generating element. The first foam-generating element may therefore be considered to be a tablet or pill, although it will be appreciated that it may be uncompressed and simply be a powder. In the solid form, the carbonate and acid do not react. The first foam-generation element 26 further comprises a stabiliser and/or thickener. Here stabilisers and/or thickeners such as lecithin and xanthan gum are used; however, any thickening or stabilising agent may be considered.

The cross-sections of each of the first and second foam-generation elements 26, 28 are preferably circular so as to correspond to the cross-section of the flexible mixing chamber.

Referring in particular to Figure 11, the second foam-generation element 28 preferably comprises a container 68 of liquid. For example, here the second container 68 comprises a container 68 of water, although other liquids may be considered. The water may include a colourant which may assist with visual identification thereof. The container 68 is hollow, sealed and preferably has curvate ends, for example here a longitudinal cross-section of the container 68 has a stadium shape. However, other shapes may be considered, such as a ball shape. In fact, a container 68 as such may not be used and the liquid and the first foam-generation element 26 may be separated or segregated by a planar seal.

The container 68 is preferably flexible, and may be formed from a thermoplastic elastomer although other flexible materials may be considered as before. In the instance of a flexible container, the container 68 preferably includes a hole 70 therein, the hole 70 allowing for the liquid to be ejected therefrom when the container 68 is squeezed. The hole 70 is preferably small, such that when pressure is not applied the surface tension of the liquid maintains the liquid in the container 68. For example, the diameter of the hole 70 may be between 0 mm and 1 mm, and may more preferably be substantially 0.5 mm, for example being 0.45 mm. The flexible container 68 with a hole 70 therein is preferably referred to as a squeeze ball. The hole 70 is ideally in an end of the container and is oriented so as to face the first foam-generation element 26.

However, the container 68 may not be flexible and may be rigid. In the instance of a rigid or inflexible container 68, at least part of the container 68 is preferably frangible via manual pressure applied thereto and may be burstable or crushable. Breaking of at least part of the container 68 may release fluid therefrom. In the instance that the container 68 is rigid, the hole 70 therein may still be present, although it will be appreciated that this may not be necessary.

Although the liquid preferably comprises, consists or consists essentially of water, it will be appreciated that the liquid may instead be an acidic solution or a carbonate solution, and the first foam-generation element 26 may lack the acid or the carbonate.

The first and/or second foam-generation elements 26, 28 may include additional components which a user may wish to consume or inhale. In particular, the first and/or second foam-generation elements 26, 28 could include flavourings to improve a user-experience. Further additional components are discussed below.

For example, the first and/or second foam-generation elements 26, 28 may include pharmaceuticals, medicines and/or dietary supplements. This may include vitamins, exercise supplements such as pre-workout compositions, branched-chain amino acids, creatine, other supplements usually taken during a workout for endurance, over-the-counter style medicines such as cough or pain remedies and/or erectile dysfunction medicine such as sildenafil. Compounds which are taken in small doses to be ingested as and when needed are of particular relevance.

The first and/or second foam-generation elements 26, 28 may include a food or beverage substance, or a compound thereof. This may include flavourings or components of soft drinks, for example electrolyte type sports drinks or iced tea, both of which can conventionally be found in powdered form. Alcoholic drinks may be considered, and in this case alcohol could be used as the liquid in the container 68. Flavourings or components of confectionary or desserts may also be considered, which may allow a user to experience a confectionary or dessert without consuming the number of calories typically associated therewith. The first and/or second foam-generation elements 26, 28 may include caffeine, for example a total caffeine content of the first and/or second foam-generation elements 26, 28 being substantially equivalent to a conventional espresso shot.

The first and/or second foam-generation elements 26, 28 may include nicotine and therefore may provide an alternative to smoking. The first and/or second foam-generation elements 26, 28 may also include other components of tobacco or tobacco itself.

The first and/or second foam-generation elements 26, 28 could include cannabis or components of cannabis, for example including tetrahydrocannabinol (THC) and/or cannabidiol (CBD) to provide psychoactive and/or medicinal effects as desired. Although cannabis is described in this specification, in view of the different legal and moral statuses of cannabis worldwide, it will be appreciated that reference to cannabis and/or components thereof may be deleted from the specification if necessary.

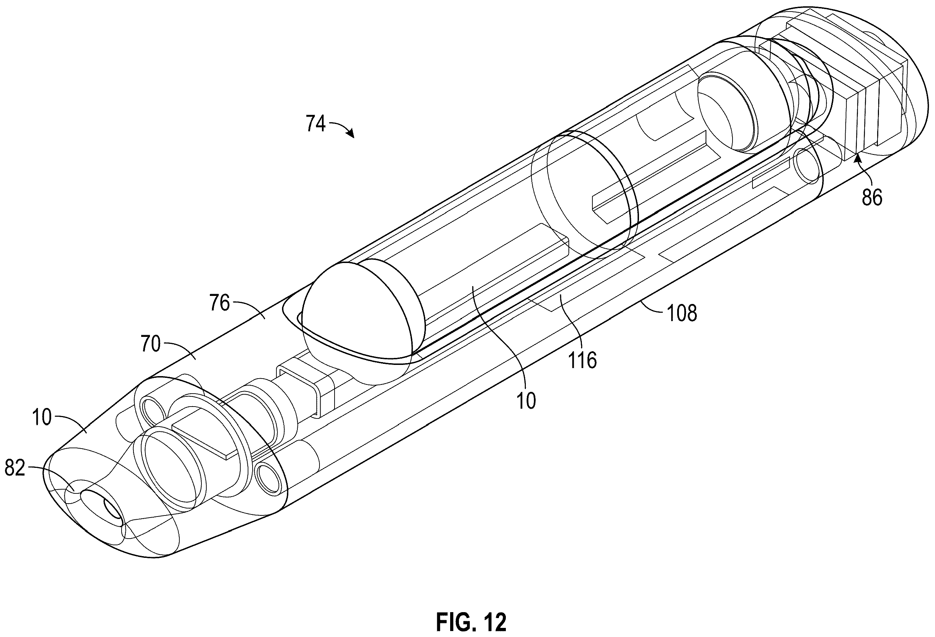

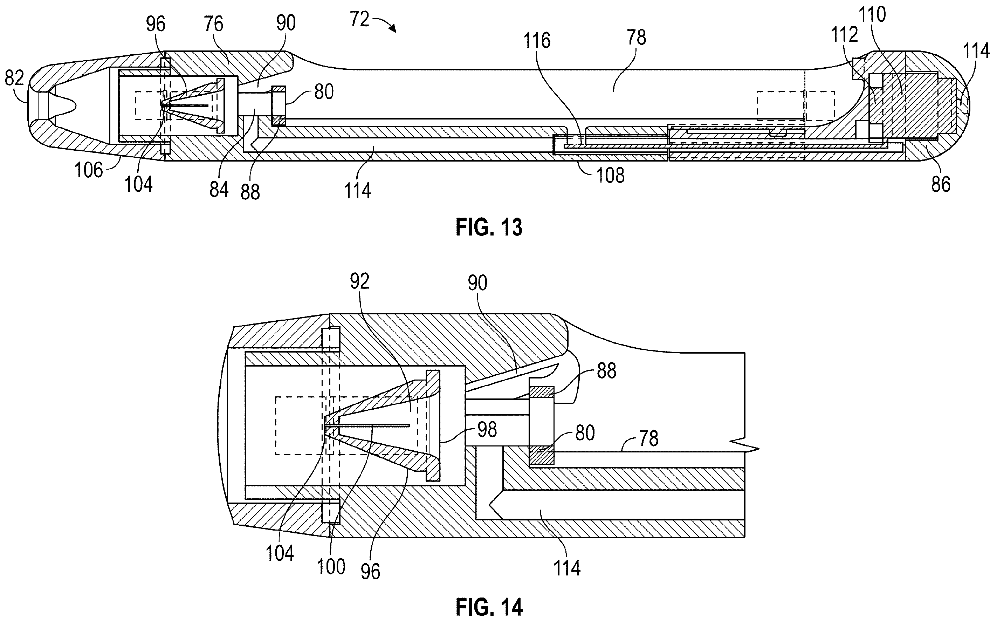

As shown in Figures 12 and 13, the cartridge 10 is received in a second embodiment of a carrier 72 or holder to form the second embodiment of an inhalation device 74. The second embodiment of the carrier 72 comprises a carrier body 76, a cartridge receiving portion 78 for receiving the cartridge 10 at or in the carrier body 76, a carrier foam inlet 80, a carrier outlet 82 and a passageway 84 or conduit between the carrier foam inlet 80 and the carrier outlet 82 through the carrier body 76.

The cartridge receiving portion 78 is here a hole 44 or recess in the carrier body 76 and is sized to captively hold the cartridge 10. A rear part 86 of the carrier body 76 is removably attachable to the remainder of the carrier body 76 so as to allow insertion of the cartridge 10.

The outlet opening 54 of the cartridge 10 is fluidly communicable with the carrier foam inlet 80 of the carrier 72, and may be insertable therein. The carrier foam inlet 80 includes a seal 88 for sealing the outlet opening 54 of the cartridge 10 thereto. The seal 88 is preferably an O-ring, and may be elastomeric.

At or adjacent to the carrier foam inlet 80 is a carrier air inlet 90. The air inlet 90 allows for the addition of air to or the entrainment of air with the foam. The air inlet 90 is preferably a channel or conduit through the carrier body 76 to the exterior of the device. Altematively, the air inlet 90 might feasibly be or communicable with a void in the carrier body 76.

A venturi tube 92 is preferably fluidly communicable with the carrier foam inlet 80 and is preferably also fluidly communicable with the carrier air inlet 90. The venturi tube 92 has a rear opening which is communicable with both inlets 80, 92. A passageway through the tube 92 may taper to increase a speed of flow of fluid therethrough, although it will be appreciated that this may not be strictly necessary. A head portion of the venturi tube 92 has a tapered and preferably frusto-conical shape. The venturi tube 92 may help to mix the foam and the air together.

The carrier 72 further includes a one-way valve 96 between the carrier foam inlet 80 and a carrier outlet 82. The one-way valve 96 is arranged to prevent a user from exhaling into the cartridge 10 or device and disrupting a foam distribution therein. The one-way valve 96 is preferably a duck-bill valve 96. The duck-bill valve 96 comprises a flexible material and is conical or substantially conical in shape, having an opening 98 at a base. The valve 96 includes a cut 100 part of the way along a body of the valve 96 from the tip or forward part of valve 96. The cut 100 allows for fluid to flow from the base and out of the tip. This is since the portions of the body demarcated by the cut 100 separate from each other to create a forward opening in the valve 96 when under outward-pressure from the interior of the of the body of the valve. When a tip portion 104 is under inward pressure from the exterior of the body of the valve, the portions of the body demarcated by the cut 100 remain in contact to prevent fluid flow therethrough.

The tapered end of the venturi tube 92 is preferably received through the opening 98 in the base of the one-way valve 96.

A front part 106 of the carrier body 76 is preferably removable from the remainder of the body 76 and/or a central part 108 of the carrier body 76, for example to assist with cleaning. The front part 106 could include the one-way valve 96 and/or the venturi tube 92, although this is not the case as shown. The front part 106 and/or the central part 108 of the carrier body 76 may include magnetic elements to allow for convenient detachable attachment therebetween. The carrier 72 may also include a sealing element between the front part 106 and the central part 108.

Referring to Figures 14 to 16 in particular, the second embodiment inhalation device 74 may also include an indication or signalling means or element 110 for indicating or signalling when a user is inhaling from the inhalation device 74. The indication element 110 may include a pressure or flow sensor 112 for detecting when negative pressure is applied thereto. As shown in Figure 14, a conduit 114, sub conduit or channel extends from the passageway 84 between the carrier foam inlet 80 and the carrier outlet 82 to the flow sensor 112. The flow sensor 112 is preferably positioned in or at the rear part 86 of the carrier 72. However, in some instances the pressure sensor 112 may be directly at or in the passageway.

The indication element 110 further includes a light-emitting device 116, such as a light emitting diode, which is configured to light when a negative pressure is sensed by the pressure sensor 112. The light-emitting device 116 is preferably positioned within the carrier body 76 and at or adjacent to the cartridge 10. For example, being at or adjacent to a central region of the cartridge receiving portion 78 of the carrier 72. To accommodate the light-emitting device 116 in this position, the rear carrier part 86 may include a projecting portion 118 which projects from a body portion of the rear carrier part 86 and supports the light-emitting device 116 and wiring or an electrical track. The central part 108 of the carrier 72 may include a hole for receiving the projecting portion 118.

The light-emitting device 116 is electrically, electronically or communicatively connected with the pressure sensor 112. The indication element 110 may include a controller configured to determine when the flow sensor 112 measures a negative pressure and then instruct the light-emitting device 116 to illuminate.

The indication element 110 preferably includes a battery to power the light-emitting device 116, pressure sensor 112, and controller if present. The battery is preferably a rechargeable battery and the carrier 72 includes an electrical terminal 120 for recharging the battery.

It will be appreciated that the indication element 110 may not be essential for the device to dispense foam and so may not be included. As such, for the purpose of dispensing foam, the second embodiment of the device is devoid of electrical components and/or is non-electrically-energisable. Of course, this contrasts to the first embodiment of the device which requires electrical energisation for producing foam.

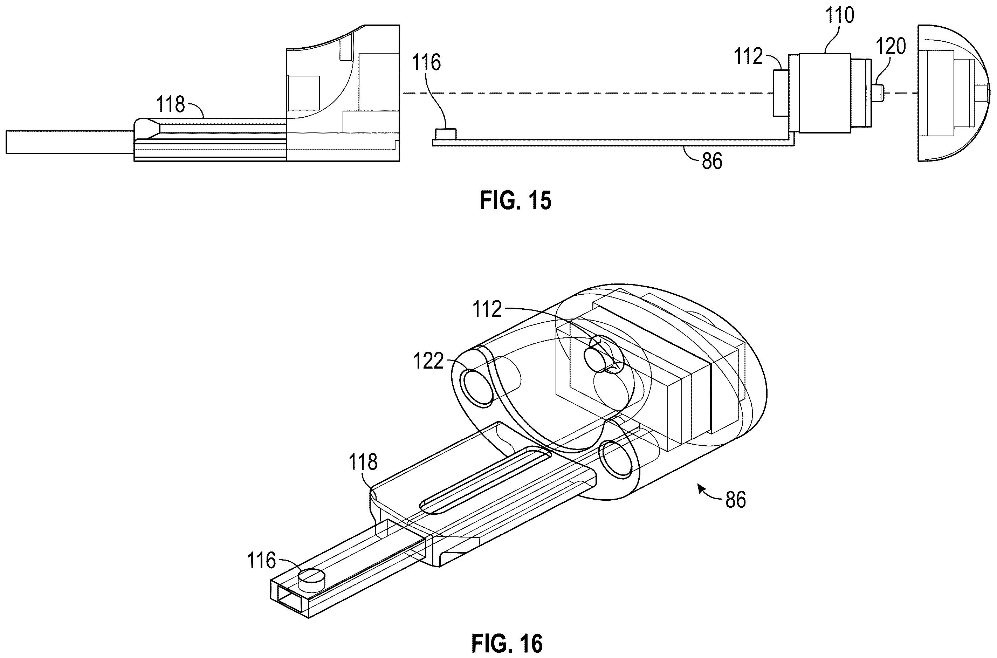

Referring in particular to Figure 16, the rear carrier part 86 preferably includes part of the cartridge receiving portion 78. The rear carrier part 86 may include at least one magnetic element 122 for releasably coupling with magnetic elements on the central part 108 of the carrier body 76. This may allow for the rear part 86 to be conveniently detachably attachable to the central part 108.

In use, the cartridge receiving portion 78 is opened by removing the rear carrier part 86. The cartridge 10 is positioned in the cartridge receiving portion 78 and the cartridge receiving portion 78 is closed by reattaching the rear carrier part 86. When in the cartridge receiving portion 78, the outlet opening 54 of the discharge element 18 is inserted or connected with the carrier foam inlet 80 of the carrier 72.

A user may then apply pressure to the flexible mixing chamber 12 over the second foam-generation element 28. The flexible mixing chamber 12 thus deforms and causes pressure to be applied to the second foam generation element 28 which causes liquid to be ejected or dispensed from the container 68 via the hole 44. The protrusion 48 of the partitioning element 16 may assist with ejecting liquid from the container 68, since it may limit the container 68 from deforming in a direction towards the partitioning element 16. A point pressure applied by the protrusion 48 may cause additional pressure on the container 68 to encourage dispensation of the liquid.

The liquid causes the components or chemicals in the first foam-generation element 26 to dissolve. The carbonate and the acid are thus able to react to produce carbon dioxide and thereby effervesce. This effervescence, which would otherwise be short-lived, is stabilised into a stable foam by the stabiliser. The foam is thickened by the thickener.

Although a stabiliser for stabilising carbon dioxide bubbles is preferred for forming a stable foam, it will be appreciated that other methods may be utilised for forming a stable foam. For example, nitrogen gas may be utilised to generate a small bubble size which may be more stable than a conventional carbon dioxide bubble. Although a chemical reaction is described as producing the effervescence and foam, it will be appreciated that other methods of effervescence or foam generation may be considered. For example, a pressurised container 68 of gas may be released, or a liquid may be agitated to produce a foam.

As the foam is generated it may move out of the mixing chamber 12 and into the expansion chamber 14 where expansion of the foam is accommodated. The mixing arms of the partitioning element 16 mix the foam as it passes the partitioning element 16. This mixing is accomplished by the mixing arms generating a vortex in the foam as the foam passes through the apertures defined by the mixing arms. The mixing assists with dissolving the first foam-generation element 26 and therefore can prevent or limit powder from being inhaled.

The user may then inhale from or at the carrier outlet 82. The negative pressure or suction applied by the inhalation causes the duck-bill valve 96 to open and foam is drawn from the expansion chamber 14, along the discharge conduit 64 and into the carrier foam inlet 80. The foam is mixed with air which is drawn through the carrier air inlet 90. The foam and air mix passes through the venturi tube 92, through the open duck-bill valve 96 and into the user's mouth via the carrier outlet 82.

The user only inhales part of the foam with a single inhalation. Since the foam is stable, the foam may be inhaled multiple times over a prolonged duration. For example, the foam is stable or present for between 15 and 30 minutes and allows for a plurality of inhalations of foam, for example substantially 50 inhalations.

When the user inhales from the carrier outlet 82, this causes a negative pressure to act on the pressure sensor 112 which triggers the illumination of the light-emitting device 116. This provides an indication to the user and/or others that the device is being used.

Once the foam has been utilised or dispensed, the user may remove the cartridge 10 by detaching the rear carrier part 86. A new cartridge 10 may then be inserted into the cartridge receiving portion 78 as required.

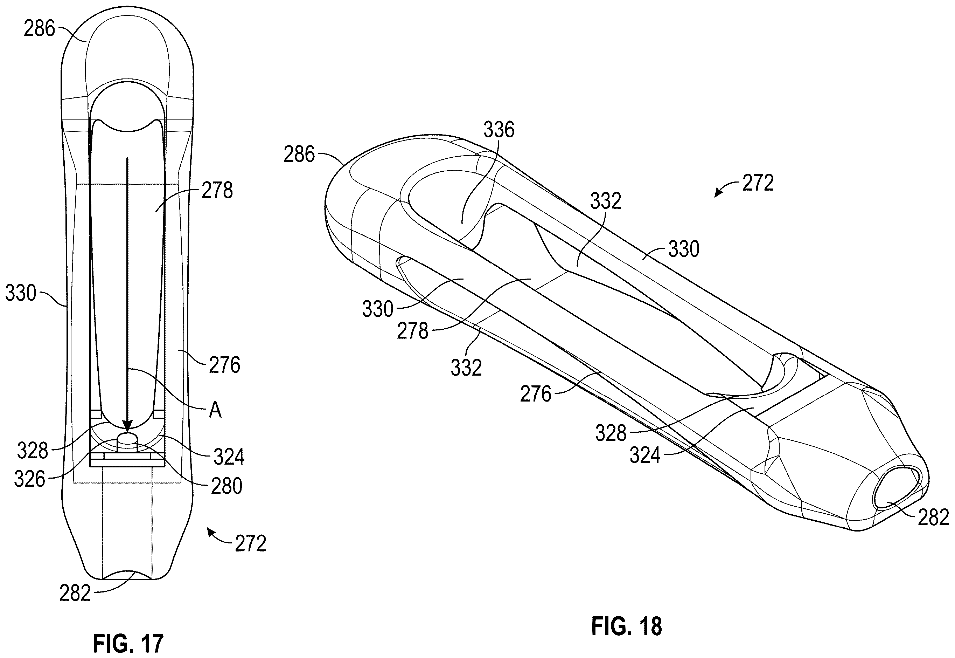

Referring now to Figures 17 to 20, there is shown a third embodiment of a carrier 272 of a stable foam inhalation device. A cartridge, such as that previously described as the second embodiment of the cartridge, is receivable in the carrier 272 to form the inhalation device. Similar or identical reference numerals are used as for the second embodiment, with 200 added. The third embodiment of the carrier 272 is preferably similar to the second embodiment 72 and has at least some similar or identical features.

The carrier body 276 of the third embodiment of the carrier 272 includes a moveable part 324 or portion at or adjacent to the cartridge receiving portion 278, and at or adjacent to the carrier foam inlet 280. An edge of the moveable part 324 may define part of the receiving portion 278. The moveable part 324 is preferably moveable so as to be received further towards the carrier outlet 282, and therefore away from a centre of the receiving portion 278. This would enlarge or elongate the receiving portion 278. The carrier body 276 may include grooves and/or a recess to accommodate the movement of the moveable part 324 towards the carrier outlet 282. However, it will be appreciated that the moveable part may be at another location at or adjacent to the receiving portion, for example being adjacent to a rear end of the carrier body. In this case, the moveable part would be moveable towards the rear end, and so away from the centre of the receiving portion.

The moveable part 324 is preferably biased towards the receiving portion 278 via a biasing means. This may be achieved via the use of a spring. For example, a coil spring may be mounted between the moveable part 324 and a remainder of the carrier body 276 which forces the moveable part 324 towards the receiving portion 278. The carrier body 276 may include a stop for the moveable part 324 to maintain the position of the moveable part 324 when the cartridge is not received in the receiving portion 278.

The moveable part 324 may include an opening 326 therein to correspond and connect with the cartridge outlet. The opening 326 in the moveable part 324 may in fact define the carrier foam inlet 280. Altematively, the cartridge outlet may be received under the moveable part with the carrier foam inlet positioned similarly under or below the moveable part.

The moveable part 324 preferably includes a concave surface 328 which corresponds to a shape of the expansion chamber of the cartridge.

The use of a moveable part 324 may have the result that a removable rear part 286, as described for the second embodiment, is not necessary, since the capsule can be inserted without removing part of the carrier body 276. However, it will be appreciated that a removable rear part may still be used if so required to enhance convenience of use.

The carrier body 276 preferably includes side walls 330 at or adjacent to the receiving portion 278. At least one of the side walls 330, and preferably both side walls 330, have an access opening 332 therein. The access openings 332 are elongate and are aligned with a longitudinal direction of the carrier body 276. The access openings 332 extend towards the movable part 324. This may allow for the cartridge, when received in the receiving portion 278, to be moved by the user's fingers towards the movable part 324, which permits release of the cartridge.

The access openings 332 are tapered, having a larger portion of the opening proximal to a rear part 286 or portion of the carrier body 276. The larger portion of the opening 332 being proximal to the rear part 286 allows for manual pressure to be more easily applied to the foam-generation elements so as to activate them and/or allows greater leverage to be applied to the cartridge from beneath to assist with removal.

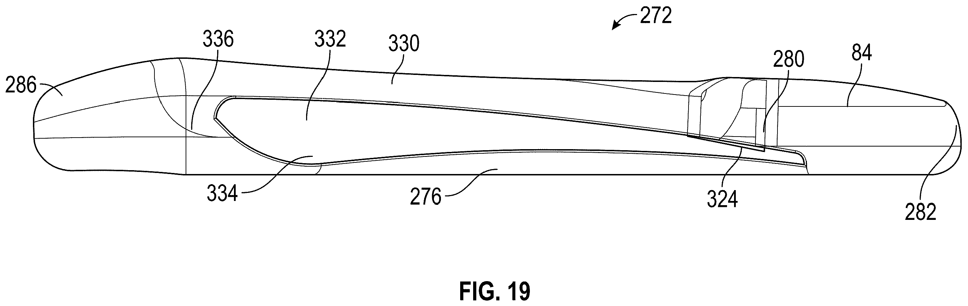

It will be appreciated that the carrier body 276 may define a space, void or gap 334 open to the access openings 332 beneath the cartridge when received in the receiving portion 278. Such a space or gap 334 may be understood with respect to Figure 19 which shows a curved portion 336 or seat where the second end of the mixing chamber may be received. It will be appreciated that the space or gap 334 may be defined below a line which extends from the seat or curved portion 336 to the carrier foam inlet 280. The gap or space 334 may assist with removal of the cartridge, for example allowing leverage to be applied underneath the cartridge. The third embodiment of the carrier 272 may be used in a similar or identical way as the second embodiment of the carrier 72 with the exception of insertion and removal of the cartridge, and the location of the application of manual pressure which is applied to the foam generation elements. The moveable part 324 is moved towards the carrier outlet 282, in the direction of block arrow A, to enlarge the receiving portion 278. The moveable part 324 may be moved via being pushed with the cartridge, for example. The cartridge is then positioned in the receiving portion 278 and the moveable part 324, under influence from the biasing means, moves towards its original position which may hold the cartridge in place.

The foam generation elements may be activated via applying manual pressure to the mixing chamber via the access openings 332. Foam is then generated and inhaled in the same or similar way as previously described.

After use, the cartridge can be moved towards the carrier outlet 282, via gripping the cartridge via the access openings 332, to move the moveable part 324 so as to enlarge the receiving portion 278. The cartridge can then be removed from the receiving portion 278, applying leverage to the cartridge via the gap 334 if required.

Although access openings are described, it will be appreciated that an upper wall or rib which defines the recess may not be included, and the opening may instead be a cut-away portion.

Although the third embodiment describes access openings and the moveable part, it will be appreciated that either or both features may be omitted.

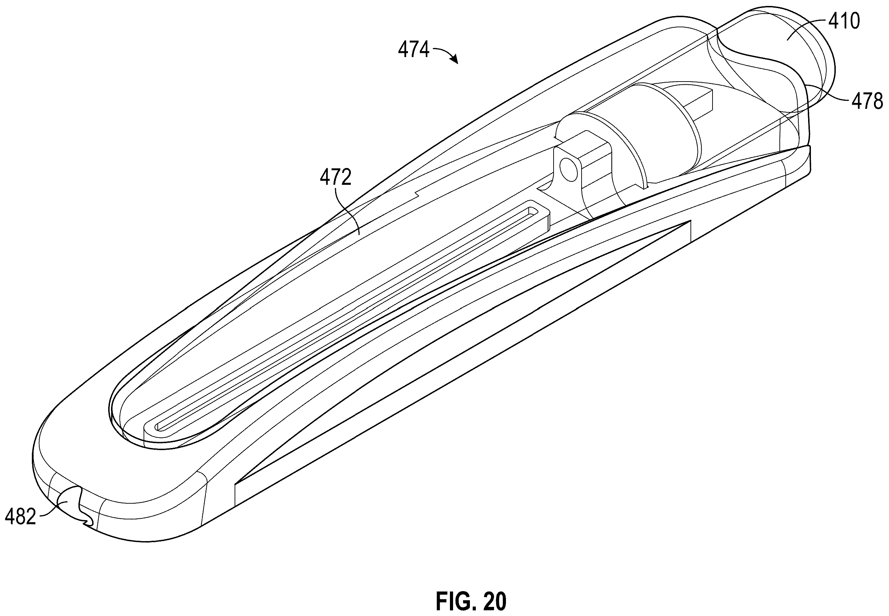

Referring now to Figures 20 to 23, there is shown a fourth embodiment of a stable-foam inhalation device 474. Similar or identical reference numerals are used as for the second or third embodiments, with 400 or 200 added respectively.

The fourth embodiment 474 has similar features to the second and third embodiments 74 and the description of those similar features of the second and third embodiments may be considered to be relevant for the fourth embodiment.

The fourth embodiment of the stable-foam inhalation device 474 comprises a fourth embodiment of a carrier 472 and a third embodiment of a cartridge 410, the cartridge 410 receivable by the carrier 474. The carrier 410 includes a cartridge-receiving portion 478 for receiving the cartridge 410, and said cartridge-receiving portion 478 may be considered to be a foam-generating-component receiving portion of the carrier 472. The carrier 472 further includes an outlet 482 for dispensing stable foam from the device 474 to be consumed or inhaled by a user.

The cartridge 410 is preferably non-electrical in that it comprises no electrical components to achieve its main function of dispensing a stable foam or stable-foam-generating components. However, the cartridge 410 may include electrical components for auxiliary functions, such as lighting or similar.

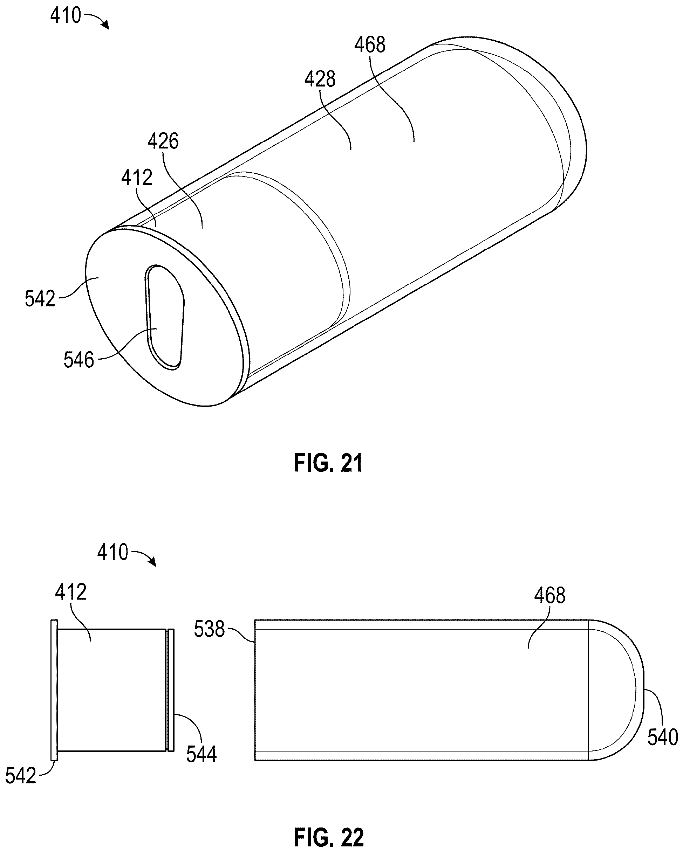

Referring to Figures 21 and 22, the cartridge 410 comprises foam-generation elements 426, 428 or components. Here there are two foam-generation elements 426, 428 or components which when combined interact to form a stable foam.

The first foam-generation element 426 comprises a powder or tablet of an acid and a carbonate as previously described. The second foam-generation element 428 comprises a liquid such as water or another solvent as previously described. However, it will be appreciated that the foam-generation elements may differ as described for previous embodiments.

The foam-generation elements 426, 428 are received in separate chambers 412, 468 or containers of the cartridge 410 with an openable barrier therebetween to prevent or limit the foam-generation elements 426, 428 from mixing or interacting until desired. The powder 426 is received in a first chamber 412, and the liquid is received in a second chamber 468. The first and second chambers 412, 468 may be considered to be a foam-generating-component receiving portion of the cartridge 410.

The second chamber 468 comprises a tube with an open end 538 and a closed end 540. At least a portion of a wall of the second chamber 468 comprises a flexible material to allow for manual application of pressure to the liquid received in the chamber 468.The first chamber 412 is preferably configured to seal or close the open end 538 of the second chamber 468. Here the first chamber 412 is receivable at least in part inside the second chamber 468, although it will be appreciated that this might not be the case. The first chamber 412 preferably includes a stop 542, which is here a flange, which has a diameter which matches or is larger than a diameter of the opening of the open end 538 of the second chamber 468 to prevent or limit over-insertion of the first chamber 412 in the second chamber 468. The flange 542 may provide bracing or support to the first chamber 412.

The first chamber 412 includes a perforable wall 544, 546 or seal at each end. The seals are each preferably planar. At a first end, which forms the openable barrier 544 between the foam-generation elements 426, 428, a first perforable wall 544 preferably comprises a foil, such as a metal foil for example aluminium foil. However, other materials, sheets or membranes may be considered for the first perforable wall. The metal foil 544 may provide a suitable seal and therefore provides a long storage life for the cartridge.

At a second end of the first chamber 412, which may form a barrier between the cartridge 410 and the carrier 472, a second perforable wall 546 may comprise a flexible sheet or membrane, such as a sheet of food-grade silicone. However, other materials, sheets or foils may be considered and it may not be flexible. It will be appreciated that a seal at the second end of the first chamber may not be strictly necessary, in the instance of the first foam-generation component being a tablet for example. However, the second perforable wall 546 may assist with providing a long storage life for the cartridge 410.

The second perforable wall 546 preferably only forms a portion of the second end of the first chamber 412, which may assist with maintaining a structural integrity of the first chamber and the cartridge since a more rigid material or construction can be used for the remainder of the second end. However, it will be appreciated that the entire second end may be perforable in some configurations. The second perforable wall 546 may be a separate sheet of material to the remainder of the second end, although alternatively the second perforable wall 546 may simply be an area of the second end with a smaller wall thickness to assist with perforation.

The second perforable wall 546 is preferably elongate, the reasons for which will be better understood hereinbelow.

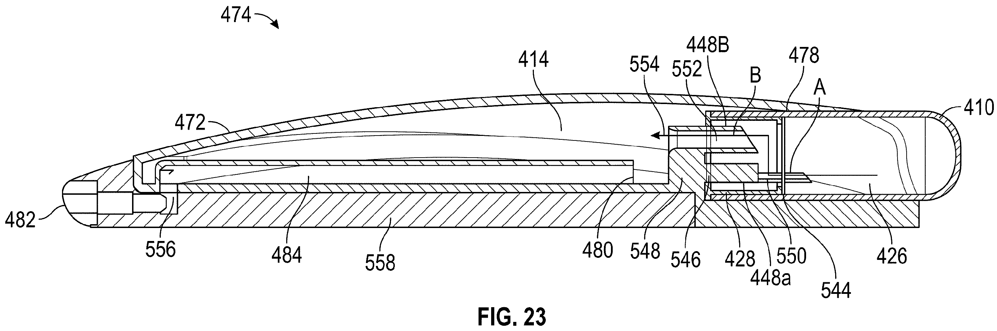

Referring now to Figure 23, the cartridge-receiving portion 478 is preferably at or adjacent to a rear of the carrier 472, and may be an opening or recess of the carrier 472. The recess 478 may be sized to receive at least a majority of the cartridge 410 inserted therein. The cartridge-receiving portion 478 includes a wall 548 to prevent over-insertion of the cartridge 410 into the carrier 472. The cartridge 410 preferably closes the opening of the cartridge-receiving portion 478 to prevent the egress of foam therefrom.

At or adjacent to the cartridge-receiving portion 478, and here projecting from said wall 548, is at least one, and preferably two, projections or protrusions 448a, 448b. The protrusions 448a, 448b are configured to perforate the first and/or second perforable walls 544, 546 of the cartridge 410 when received in the carrier 472.

The protrusions 448a, 448b may be pointed to assist with perforation of the perforable walls, however, it will be appreciated that this may not be necessary. Each protrusion 448a, 448b preferably also includes a passageway to assist with permitting fluid to flow through the perforation made in the perforable wall 544, 546 by the protrusion 448a, 448b.

The two protrusions 448a, 448b are spaced apart from each other. A first protrusion 448a projects from a lower portion of the wall 548 and a second protrusion 448b projects from an upper portion of the wall 548. The spacing of the protrusions 448a, 448b may assist with suitable mixing between the foam-generation elements or components 426, 428.

The first protrusion 448a has a longer projecting extent than that of the second protrusion 448b. As such, the first protrusion 448a is configured to extend through the first chamber 412, via a lower portion of the elongate second perforable wall 546, and through the first perforable wall 544. The first protrusion 448a defines an open-top channel 550 to prevent the perforation in the first perforable wall 544 being blocked by the first protrusion 448a.

The second protrusion 448b is configured to extend part way into the first chamber 412. The second protrusion 448b includes a bore 552 to permit foam to flow or expand therethrough. The bore 552 extends through the second perforable wall 546. The second protrusion 448b has a larger diameter than that of the first protrusion 448a, which may be beneficial for permitting the passage of foam therethrough.

The carrier 472 preferably includes an expansion chamber 414 and a conduit 484 which are preferably between the cartridge-receiving portion 478 and the outlet 482. Together, the expansion chamber 414 and conduit 484 may be considered passageway or fluid flow path between the cartridge-receiving portion 478 and the outlet 482. The expansion chamber 414 is preferably at or adjacent to the receiving portion 478 and the bore 552 of the second protrusion 448b. The bore 552 may have a widening 554 at an end which is at or adjacent to expansion chamber 414 which may encourage foam therein. The expansion chamber 414 is so called since it provides space for the foam to expand into.

The expansion chamber 414 may be transparent to allow for the foam to be visible during use. However, it will be appreciated that this may not be necessary. The expansion chamber 414 may be flexible to allow for manual manipulation thereof, although, again, it will be appreciated that this may not be necessary.

The conduit 484 preferably extends from the outlet 482 and into the expansion chamber 414. The conduit 484 preferably has an inlet opening 480 in the expansion chamber 414 for receiving foam. The inlet opening 480 of the conduit 484 is preferably off-centre relative to the expansion chamber 414 being at an in-use bottom or lower portion of the expansion chamber 414. This may allow for more of the foam in the expansion chamber 414 to be consumed, since the foam may sink towards the bottom under gravity. Additionally or alternatively, the inlet opening 480 is proximal to the cartridge-receiving portion 478 as compared to the outlet 482. Again, this may allow for more of the foam in the expansion chamber 414 to be consumed, for example, if the device is used in a declining orientation with the cartridge-receiving portion 478 lower than the outlet 482.

The conduit 484 preferably also includes a drop, step or turn 556, and as such the conduit 484 is non-linear, however it will be appreciated that this may not be necessary. The conduit 484 may include a one-way valve and/or venturi tube, as described for the preceding embodiments.

The outlet 482, or mouth-piece, is preferably cross-shaped or substantially cross-shaped, although it will be appreciated that other shapes may be considered. The outlet 482 may additionally or alternatively represent a widening of the conduit 484 which may encourage emission of foam therefrom.

The carrier may also include a holder 558 which may receive the expansion chamber 414 and define at least part of the conduit 484. The holder may 558, for example, be formed from a more rigid material than the expansion chamber 414. It will be appreciated that the fourth embodiment of the device 474 may include sensors, seals, lighting and/or sound means as previously described for the previous embodiments.

In use, the fourth embodiment of the device 474 may function in a similar or identical way as the second and third embodiments. The cartridge 410 is inserted into the cartridge-receiving portion 478. This action causes the first protrusion 448a to first perforate the second perforable wall 546, extend through the first chamber 412, and then perforate the first perforable wall 544 to open the barrier between the foam-generation elements 426, 428. Similarly, the second protrusion 448b may perforate the second perforable wall 546. The user may then squeeze an exposed end of the flexible second chamber 468 to exert pressure on the liquid to force it through the perforation and the channel 550 defined by the first protrusion 448a and into the first chamber 412. This is indicated by line A in Figure 23. In the first chamber 412, the foam-generation elements 426, 428 mix and interact to generate a stable foam which expands or flows through the bore 552 of the second protrusion 448b and into the expansion chamber 414, as indicated by arrow B. The expansion chamber 414 becomes filled or partially filled with the stable foam. The stable foam may then drawn by the user's inhalations on the outlet 482 through the conduit 484, out of the outlet 482 and into the users mouth to be inhaled or consumed. The stable foam may be consumed over multiple inhalations and/or over prolonged duration.

It is therefore possible to provide a device which produces a stable foam, as opposed to only a short-lived effervescence and/or a rapidly propelled inhalant, which allows for a user to consume or inhale a substance contained within the foam over a prolonged duration and with multiple inhalations. This provides a smoking alternative device or cigarette substitute.

The words 'comprises/comprising' and the words 'having/including' when used herein with reference to the present invention are used to specify the presence of stated features, integers, steps or components, but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

The embodiments described above are provided by way of examples only, and various other modifications will be apparent to persons skilled in the field without departing from the scope of the invention as defined herein.

### ORIGINAL PARENT CLAIMS FORMING PART OF THE DESCRIPTION AS

### PREFERRED EMBODIMENTS OF THE DIVISIONAL APPLICATION ONLY

1. A battery-operated foam inhalation device comprising:
   a cartridge (610) having a liquid for producing a foam, and a cartridge foam outlet (654);
   agitation means for agitating the liquid to produce the foam, the agitation means comprising an electric motor (762) and a battery (760) for energising the electric motor (762); and
   a carrier (672) having
      a receiving portion (678) for receiving the cartridge (610) at or in the carrier (672),
      a carrier foam inlet (680) for fluid communication with the cartridge foam outlet (654) when the cartridge (610) is received at or in the receiving portion (678),
      a carrier foam outlet (682) for dispensing the foam to a user, and
      a conduit (684) between the carrier foam inlet (680) and the carrier foam outlet (682).
2. A battery-operated foam inhalation device as claimed in claim 1, further comprising a one-way valve (96) for permitting movement of foam from the cartridge foam outlet (654) to the carrier foam outlet (682) and preventing or limiting movement of fluid from the carrier foam outlet (682) to the cartridge foam outlet (654).
3. A battery-operated foam inhalation device as claimed in claim 1 or claim 2, further comprising a pressure sensor (712) for detecting when negative pressure is applied to the carrier foam outlet (682).
4. A battery-operated foam inhalation device as claimed in claim 3, wherein the pressure sensor (712) is communicatively connected with the agitation means so that when the pressure sensor (712) detects negative pressure the agitation means is activated so as to agitate the liquid to produce foam.
5. A battery-operated foam inhalation device as claimed in any one of the preceding claims, wherein the carrier (672) further comprises a carrier air inlet (690).
6. A battery-operated foam inhalation device as claimed in claim 5, wherein the cartridge (610) comprises a cartridge air inlet (774), the carrier (672) comprises an air-flow conduit 766 from the carrier air inlet (690) for connection with the cartridge air inlet (774), and the agitation means comprises an air moving means driven by the electric motor (762) for moving air from the air inlet to the cartridge (610).
7. A battery-operated foam inhalation device as claimed in claim 6 wherein the air moving means comprises an air pump (770).
8. A battery-operated foam inhalation device as claimed in claim 6 or claim 7, wherein the cartridge (610) comprises an air-injection conduit 776 which extends from the cartridge air inlet (774) into an interior of the cartridge (610) for injecting air into the cartridge (610) via at least one opening (780) in the air-injection conduit (776).
9. A battery-operated foam inhalation device as claimed in claim 8, wherein the air-injection conduit (776) has a plurality of openings (780) therein.
10. A battery-operated foam inhalation device as claimed in claim 9, wherein there are at least five openings (780).
11. A battery-operated foam inhalation device as claimed in claim 9 or claim 10, wherein the air-injection conduit (776) is elongate and the plurality of openings (780) are spaced apart along a longitudinal extent thereof.
12. A battery-operated foam inhalation device as claimed in any one of claims 8 to 11, wherein said at least one opening (780) is closer to an end of the cartridge (610) which opposes the cartridge air inlet (774) than to the cartridge air inlet (774).
13. A battery-operated foam inhalation device as claimed in any one of claims 6 to 12, wherein each of the cartridge foam outlet (654) and the cartridge air inlet (774) comprises a seal, at least part of the conduit (684) of the carrier (672) is configured to perforate the seal of the cartridge foam outlet (654), and at least part of the air-flow conduit (766) is configured to perforate the seal of the cartridge air inlet (774).
14. A battery-operated foam inhalation device as claimed in claim 13, wherein the conduit (684) of the carrier (672) and the air-flow conduit (766) project into the receiving portion (678) so as to permit perforation of the cartridge foam outlet (654) and the cartridge air inlet (774) respectively.
15. A battery-operated foam inhalation device as claimed in claim 13 or claim 14, wherein each seal is flexible so as to seal the cartridge foam outlet (654) to the conduit (684) of the carrier (672), and so as to seal the cartridge air inlet (774) to the air-flow conduit (766).
16. A battery-operated foam inhalation device as claimed in any one of the preceding claims, further comprising a cartridge-presence sensor configured to detect when the cartridge (610) is received in the cartridge receiving portion (678), the agitating means configured to operate only when the cartridge-presence sensor detects the cartridge (610) in the cartridge receiving portion (678).
17. A battery-operated foam inhalation device as claimed in any one of the preceding claims, wherein the liquid comprises at least one of nicotine, a component of cannabis, medicines and/or dietary supplements, and a food or drink substance.
18. A battery-operated foam inhalation device as claimed in any one of the preceding claims, wherein the device is elongate and has a length of 150 mm or less and a width of 50 mm or less.
19. A foam drawing device for a user to draw foam therefrom with breath of the user, the foam drawing device comprising:
   a container (610) for containing a liquid for producing a foam, the container (610) having a container foam outlet (654) for emitting foam and a container air inlet (774) for receiving air;
   an electrically energisable air moving means (762, 770) for moving air to the container air inlet (774) from an exterior of the device;
   a power source (760) for powering the electrically energisable air moving means (762, 770);
   a mouthpiece (764) fluidly communicated with the foam outlet (654) for a user to draw foam from;
   a pressure sensor (712) fluidly communicated with the mouthpiece (764) for detecting when negative pressure is applied to the mouthpiece (764);
   a processor (772) configured to operate the electrically energisable air moving means (762, 770) when the pressure sensor (712) detects when negative pressure is applied to the mouthpiece.
20. A foam inhalation device comprising:
   a cartridge (610) having a liquid for producing a foam, and a cartridge foam outlet (654) (54);
   an agitation means for agitating the liquid to produce foam; and
   a carrier (672) having
      a receiving portion (678) for receiving the cartridge (610) at or in the carrier (72),
      a carrier foam inlet (680) for fluid communication with the foam outlet (54) of the cartridge (610) when the cartridge (610) is received at or in the receiving portion (678),
      a carrier foam outlet (682) for dispensing the foam to a user,
      a conduit (684) between the carrier foam inlet (680) and the carrier foam outlet (54); and
      a carrier air inlet (690).

## Claims

1. A stable-foam consumption device (10) for dispensing a stable foam produced by effervescence to be consumed by a user over a prolonged duration, the consumption device (10) comprising:
a foam-generating-component receiving portion (30, 32);
effervescent stable-foam-generating components (26, 28) for generating the stable foam, wherein said components (26, 28) are received in the foam-generating-component receiving portion (30, 32), include nicotine and a stabiliser or thickener, and are configured to generate the stable foam via effervescence; and
an outlet (54) for dispensing the stable foam to the user.

2. A stable-foam consumption device (10) as claimed in claim 1, wherein the stable-foam-generating components (26, 28) comprise an acid and a carbonate.

3. A stable-foam consumption device (10) as claimed in claim 2, wherein the acid and carbonate comprise at least any one of citric acid, sodium bicarbonate, calcium carbonate, and tartaric acid

4. A stable-foam consumption device (10) as claimed in claim 2 or claim 3, wherein at least one of the acid and carbonate is a powder.

5. A stable-foam consumption device (10) as claimed in any one of the preceding claims, wherein the stabiliser or thickener comprises at least any one of lectin, lecithin and xanthan gum.

6. A stable-foam consumption device (10) as claimed in any one of the preceding claims, wherein the foam-generating-component receiving portion (30, 32) comprises a flexible wall.

7. A stable-foam consumption device (10) as claimed in any one of the preceding claims, wherein the outlet (54) is configured to be at or in the user's mouth.
